# EUROPEAN PATENT APPLICATION

(11) **EP 4 059 528 A1**
(43) Date of publication of application: **21.09.2022**
(21) Application number: 20887308.3
(22) Date of filing: 21.10.2020
(51) Int. Cl.: A61L 101/22, B05B 17/06, A61L 2/18

(54) **DECONTAMINATION SYSTEM**

(30) Priority: 15.11.2019 JP 2019206606; 28.04.2020 JP 2020078816
(71) Applicant: Airex Co., Ltd., Nagoya-shi Aichi 453-0015 (JP)
(72) Inventor: KAWASAKI, Koji, Nagoya-shi Aichi 453-0015 (JP); SOU, Gun, Nagoya-shi Aichi 453-0015 (JP); KITANO, Tsukasa, Nagoya-shi Aichi 453-0015 (JP); GUO, Zhiqiang, Nagoya-shi Aichi 453-0015 (JP); FUTAMURA, Haruka, Nagoya-shi Aichi 453-0015 (JP); YAZAKI, Yukihiro, Nagoya-shi Aichi 453-0015 (JP); KAKUDA, Daisuke, Nagoya-shi Aichi 453-0015 (JP); MASUDOME, Jun, Nagoya-shi Aichi 453-0015 (JP)
(74) Representative: Lederer & Keller Patentanwälte Partnerschaft mbB
(86) International application number: PCT/JP2020/039556
(87) International publication number: WO 2021/095465

(57) **Abstract**

The present invention achieves a decontamination effect with a proper amount of hydrogen peroxide mist supplied to a room to be decontaminated by further refining a hydrogen peroxide mist supplied to the room to be decontaminated for dispersion/diffusion, and reducing the duration of operations such as aeration to achieve more efficient decontamination works.

The decontamination system includes a mist generation means, a mist discharge port, and a mist dispersion/diffusion means. The mist generation means converts a decontamination liquid into a mist to generate a mist for decontamination. The mist discharge port is opened at an upper portion on an internal side wall surface of a room to be decontaminated to discharge a mist for decontamination into the inside of the room to be decontaminated. The mist dispersion/diffusion means generates sound flows by an ultrasound in the vertical direction from a plate surface of a vibration plate provided adjacent to a lower portion of the mist discharge port on the internal side wall surface of the room to be decontaminated or adj acent to a lower portion on a side surface in a mist discharge direction, and the mist for decontamination is pressed by acoustic radiation pressure backward or laterally in intermittent operation or stronger/weaker operation.

## Description

### TECHNICAL FIELD

The present invention relates to a decontamination system for decontaminating an indoor working area such as not only an isolator, a RABS, and a clean room, but also a passing room and a pass box associated therewith by generating a mist for decontamination in the working area.

### BACKGROUND ART

In manufacturing settings for pharmaceutical or food products or in the clinical environment such as operating rooms, the indoor working area must inevitably be kept sterile. Particularly in cases where clean rooms as a working chamber for manufacturing pharmaceutical products are decontaminated, advanced decontamination validation needs to be accomplished in accordance with Good Manufacturing Practice (GMP).

In recent years, hydrogen peroxide gas has widely been used to decontaminate a working chamber requiring sterile environment (hereinafter referred to as a "room to be decontaminated"). Advantageously, hydrogen peroxide gas has a strong sterilization effect, and is inexpensively available and effectively utilized as an environmentally-friendly decontamination gas that is ultimately resolved into oxygen and water.

In addition, the following patent document 1 describes that the decontamination effect by hydrogen peroxide is provided by a condensed film of a hydrogen peroxide solution that condenses on the surface of an object to be decontaminated. Accordingly, in order to achieve the decontamination effect in a room to be decontaminated, hydrogen peroxide gas may be supplied in large quantities to make thick or in a higher concentration the resulting condensed film composed of a hydrogen peroxide solution.

In fact, the supply of an excessive amount of hydrogen peroxide gas to a room to be decontaminated causes extreme condensation, and the resulting condensed film from a high concentration of hydrogen peroxide solution disadvantageously corrodes production equipment, precise measuring equipment and wall surfaces and other portions of a room to be contaminated installed inside the room to be contaminated. After a decontamination work using hydrogen peroxide gas, aeration is performed with clean air to remove the residual hydrogen peroxide gas and condensed film inside the room to be decontaminated. However, the supply of such an excessive amount of hydrogen peroxide gas is problematic due to longer duration required in the aeration operation for removing a high concentration of condensed film of a hydrogen peroxide solution generated on wall surfaces and other portions of the room to be decontaminated.

Meanwhile, the supply of a small amount of hydrogen peroxide gas to a room to be decontaminated unfortunately causes insufficient condensation, resulting in unsatisfactory decontamination effects. A feasible solution to this problem is to replace hydrogen peroxide gas with a hydrogen peroxide solution in the form of a mist (hereinafter referred to as "hydrogen peroxide mist") to be supplied to a room to be decontaminated.

### CITATION LIST

### PATENT LITERATURE

Patent Document 1: JP-A-61-004543

### SUMMARY OF INVENTION

### TECHNICAL PROBLEM

However, hydrogen peroxide mists in the form of a liquid fine particle tend to be less dispersed and diffused than gaseous hydrogen peroxide gas. As a result, the supply of a proper and small amount of hydrogen peroxide mist to a room to be decontaminated can fail to uniformly disperse and diffuse the hydrogen peroxide mist inside the room to be decontaminated, causing insufficient decontamination. On the other hand, the supply of an excessive amount of hydrogen peroxide mist to the room to be decontaminated generates large particles, which unfortunately fall onto the floor of a room to be decontaminated for condensation. The resulting condensation can corrode wall surfaces of a room to be decontaminated. Another problem with condensation occurrence is longer aeration duration for removing condensation.

Thus, the present invention was made in view of the situation to solve the problems, and has an object to provide a decontamination system capable of achieving a decontamination effect with a proper amount of hydrogen peroxide mist supplied to a room to be decontaminated by further refining a hydrogen peroxide mist supplied to the inside of the room to be decontaminated for dispersion and diffusion, and reducing the duration of operations such as aeration to achieve more efficient decontamination works.

### SOLUTION TO PROBLEM

To solve the aforementioned problem, inventors of the present invention have carried out an extended investigation to find that a hydrogen peroxide mist supplied to the inside of a room to be decontaminated is subjected to ultrasonic sound flow to be further refined, and the hydrogen peroxide mist is pressed by acoustic radiation pressure for dispersion and diffusion. Based on that technique, the present invention was accomplished.

Specifically, a decontamination system (100 to 500) according to the present invention is, according to description in claim 1, includes
a mist generation means (M1 to M11) for generating a mist for decontamination by converting a decontamination liquid into a mist, a mist discharge port (X1 to X4) for discharging the mist for decontamination into the inside of a room to be decontaminated (R1 to R4), and a mist dispersion/diffusion means (D1 to D5) for dispersing and diffusing the mist for decontamination discharged, characterized in that
the mist discharge port is opened on an internal side wall surface of the room to be decontaminated to discharge the mist for decontamination into the inside of the room to be decontaminated, and
the mist dispersion/diffusion means includes a vibration plate disposed adjacent to a lower portion of the mist discharge port on an internal side wall surface of the room to be decontaminated or adjacent to a side surface in a mist discharge direction, the vibration plate is subjected to ultrasonic vibration to generate sound flows from a plate surface by an ultrasound in the vertical direction, and the mist for decontamination discharged from the mist discharge port is pressed by acoustic radiation pressure backward or laterally in stationary operation, intermittent operation or stronger/weaker operation to thoroughly disperse and diffuse the mist for decontamination entirely inside the room to be decontaminated.

Moreover, the present invention is, according to description in claim 2, the decontamination system according to claim 1, characterized in that
the mist generation means includes a decontamination liquid supply unit (20) for storing a decontamination liquid and supplying the same through a decontamination liquid supply pipe (LL2) and a compressed air supply device (10) for generating compressed air and supplying the same through an air supply pipe (AL2) to generate the mist for decontamination from the supplied decontamination liquid and compressed air.

Moreover, the present invention is, according to description in claim 3, the decontamination system according to claim 1, characterized in that
the mist generation means includes a primary mist generation means (M1, M3, M4, M7) and a secondary mist generation means (M2, M5, M6, M8 to M11),
the primary mist generation means includes a decontamination liquid supply unit (20) for storing a decontamination liquid and supplying the same through a decontamination liquid supply pipe (LL1, LL3 to LL5) and a compressed air supply device (10) for generating compressed air and supplying the same through an air supply pipe (AL1, AL3 to AL5) to generate a primary mist from the supplied decontamination liquid and compressed air and supply the same to the secondary mist generation means through a primary mist supply pipe (ML1 to ML4),
the secondary mist generation means includes a primary mist receiving container (MR1 to MR5) for subjecting the primary mist supplied to gas-liquid separation and an ultrasonic atomizer (A1 to A5), and the decontamination liquid subjected to gas-liquid separation is converted into a fine, atomized secondary mist to be supplied to the mist discharge port, and
the mist discharge port discharges the secondary mist into the inside of the room to be decontaminated as the mist for decontamination.

Moreover, the present invention is, according to description in claim 4, the decontamination system according to any one of claims 1 to 3, characterized in that
the mist for decontamination supplied to the inside of the room to be decontaminated is further refined by ultrasonic vibration generated from the ultrasonic vibration plate.

Moreover, the present invention is, according to description in claim 5, the decontamination system according to claim 3, characterized in that
the primary mist generation means, which includes the decontamination liquid supply unit and the compressed air supply device, is shared by a plurality of rooms to be decontaminated, and
each of the rooms to be decontaminated includes the secondary mist generation means, the mist discharge port and the mist dispersion/diffusion means.

Furthermore, the present invention is, according to description in claim 6, the decontamination system according to claim 5, characterized in that
the decontamination liquid supply unit, the compressed air supply device and the primary mist generation means are arranged so as to be separate from the rooms to be decontaminated through the primary mist supply pipe,
the secondary mist generation means is arranged adjacent to a corresponding room to be decontaminated or indoor through the primary mist supply pipe, whereby
the conveyance distance of the primary mist supply pipe to each room to be decontaminated is longer than the conveyance distance of the decontamination liquid supply pipe corresponding to the room to be decontaminated.

Moreover, the present invention is, according to description in claim 7, the decontamination system according to any one of claims 3, 5, and 6, characterized in that
the ultrasonic atomizer includes a piezoelectric vibrator (A1-3, A2-3) and a perforated vibration plate (A1-2, A2-2) provided with a plurality of micropores for atomizing the decontamination liquid subjected to gas-liquid separation by vibration of the piezoelectric vibrator, the micropores passing through the perforated vibration plate between the front surface and the back surface thereof,
the ultrasonic atomizer is disposed such that the front surface of the perforated vibration plate faces the inside of the room to be decontaminated as the mist discharge port and the rear surface faces the inside of the primary mist receiving container, and
the primary mist supplied to the primary mist receiving container is ejected from the primary mist supply pipe onto the rear surface of the perforated vibration plate to be subjected to gas-liquid separation, and is atomized when the separated decontamination liquid moves from the rear surface to the front surface of the perforated vibration plate to be discharged into the inside of the room to be decontaminated with the front surface serving as the mist discharge port.

Moreover, the present invention is, according to description in claim 8, the decontamination system according to any one of claims 3, 5, and 6, characterized in that
the ultrasonic atomizer includes a piezoelectric vibrator (A1-3, A2-3) and a perforated vibration plate (A1-2, A2-2) provided with a plurality of micropores for atomizing the decontamination liquid subjected to gas-liquid separation by vibration of the piezoelectric vibrator, the micropores passing through the perforated vibration plate between the front surface and the back surface thereof,
the ultrasonic atomizer is disposed such that the front surface of the perforated vibration plate faces the inside of the room to be decontaminated as the mist discharge port and the rear surface faces a liquid pool (MR1-2, MR2-2) provided at an internal lower end portion of the primary mist receiving container, and
the primary mist supplied to the primary mist receiving container is discharged from the primary mist supply pipe into the inside of the primary mist receiving container to be subjected to gas-liquid separation, and is atomized after the separated decontamination liquid is collected at the liquid pool of the primary mist receiving container and moves from the rear surface to the front surface of the perforated vibration plate to be discharged into the inside of the room to be decontaminated with the front surface serving as the mist discharge port.

### ADVANTAGEOUS EFFECTS OF INVENTION

According to the above configuration, the decontamination system of the present invention includes a mist supply means, a mist discharge port and a mist dispersion/diffusion means. The mist generation means converts a decontamination liquid into a mist to generate a mist for decontamination. The mist discharge port is opened on an internal side wall surface of a room to be decontaminated to discharge a mist for decontamination into the inside of the room to be decontaminated. The mist dispersion/diffusion means subjects to ultrasonic vibration a vibration plate provided adjacent to a lower portion of the mist discharge port on an internal side wall surface of the room to be decontaminated or adjacent to a side surface in a mist discharge direction to generate sound flows, and the mist for decontamination discharged from the mist discharge port is pressed by acoustic radiation pressure backward or laterally in intermittent operation or stronger/weaker operation. Accordingly, the mist for decontamination can thoroughly be dispersed and diffused entirely inside the room to be decontaminated.

According to the above configuration, the mist generation means includes a decontamination liquid supply unit and a compressed air supply device. The decontamination liquid supply unit stores a decontamination liquid and supplies the same through a decontamination liquid supply pipe. The compressed air supply device generates compressed air and supplies the same through an air supply pipe. Herein, the mist generation means generates a mist for decontamination from the supplied decontamination liquid and compressed air. Accordingly, the above operational advantage can more specifically be provided.

According to the above configuration, the decontamination system is composed of a primary mist generation means and a secondary mist generation means . The primary mist generation means includes a decontamination liquid supply unit and a compressed air supply device. The decontamination liquid supply unit supplies the stored decontamination liquid through a decontamination liquid supply pipe. The compressed air supply device supplies the generated compressed air through an air supply pipe. Herein, the primary mist generation means generates a primary mist from the supplied decontamination liquid and compressed air and supplies the same to the secondary mist generation means through a primary mist supply pipe.

The secondary mist generation means includes a primary mist receiving container and an ultrasonic atomizer. The primary mist receiving container subjects the supplied primary mist to gas-liquid separation. The ultrasonic atomizer converts the decontamination liquid subjected to gas-liquid separation into a fine, atomized secondary mist and supplies the same into a mist discharge port. The mist discharge port discharges the secondary mist into the inside of the room to be decontaminated as a mist for decontamination. Accordingly, the above operational advantage can more efficiently be provided.

According to the above configuration, the mist for decontamination supplied to the inside of the room to be decontaminated is further refined by ultrasonic vibration generated from a vibration plate. Accordingly, the above operational advantage can more efficiently be provided.

According to the above configuration, in the decontamination system, the primary mist generation means, which includes the decontamination liquid supply unit and the compressed air supply device, is shared by a plurality of rooms to be decontaminated. Meanwhile, each room to be decontaminated includes a secondary mist generation means, a mist discharge port and a mist dispersion/diffusion means .

According to the above configuration, the decontamination liquid supply unit, the compressed air supply device and the primary mist generation means are arranged so as to be separate from the rooms to be decontaminated through the primary mist supply pipe. Meanwhile, the secondary mist generation means is arranged adjacent to a corresponding room to be decontaminated or indoor through the primary mist supply pipe. Accordingly, the conveyance distance of the primary mist supply pipe to a room to be decontaminated is longer than the conveyance distance of the decontamination liquid supply pipe corresponding to the room to be decontaminated.

According to the above configuration, the ultrasonic atomizer includes a piezoelectric vibrator and a perforated vibration plate provided with a plurality of micropores for atomizing the decontamination liquid subjected to gas-liquid separation by vibration of the piezoelectric vibrator, the micropores passing through the perforated vibration plate between the front surface and the back surface thereof. The ultrasonic atomizer is disposed such that the front surface of the perforated vibration plate faces the inside of the room to be decontaminated as the mist discharge port and the rear surface faces the inside of the primary mist receiving container. In this state, the primary mist supplied to the primary mist receiving container is ejected from the primary mist supply pipe onto the rear surface of the perforated vibration plate to be subjected to gas-liquid separation. The separated decontamination liquid is atomized when it moves from the rear surface to the front surface of the perforated vibration plate to be discharged from into the inside of the room to be decontaminated with the front surface serving as the mist discharge port.

According to the above configuration, the ultrasonic atomizer includes a piezoelectric vibrator and a perforated vibration plate provided with a plurality of micropores for atomizing the decontamination liquid subjected to gas-liquid separation by vibration of the piezoelectric vibrator, the micropores passing through the perforated vibration plate between the front surface and the back surface thereof. The ultrasonic atomizer is disposed such that the front surface of the perforated vibration plate faces the inside of the room to be decontaminated as the mist discharge port and the rear surface faces a liquid pool provided at an internal lower end portion of the primary mist receiving container. In this state, the primary mist supplied to the primary mist receiving container is discharged from the primary mist supply pipe into the inside of the primary mist receiving container to be subjected to gas-liquid separation, and is atomized after the separated decontamination liquid is collected at the liquid pool of the primary mist receiving container and moves from the rear surface to the front surface of the perforated vibration plate to be discharged into the inside of the room to be decontaminated with the front surface serving as the mist discharge port.

Thus, the present invention can provide a decontamination system capable of achieving a decontamination effect with a proper amount of hydrogen peroxide mist supplied to a room to be decontaminated by further refining a hydrogen peroxide mist supplied to the inside of the room to be decontaminated for dispersion/diffusion, and reducing the duration of operations such as aeration to achieve more efficient decontamination works.

### BRIEF DESCRIPTION OF DRAWINGS

FIG. 1 is a schematic block diagram illustrating a decontamination system according to a first embodiment of the present invention;
FIG. 2 is an external perspective view of a secondary mist generation device used in the first embodiment;
FIG. 3(A) is a front view of the secondary mist generation device in FIG. 2 seen from the side of a room to be decontaminated and FIG. 3(B) is a cross-sectional view of the secondary mist generation device seen from the right side;
FIG. 4 is an external perspective view of a mist dispersion/diffusion device used in the first embodiment;
FIG. 5 is an image diagram illustrating the state of a hydrogen peroxide mist dispersed and diffused in the decontamination system in FIG. 1;
FIG. 6 is a schematic block diagram illustrating a decontamination system according to a second embodiment of the present invention;
FIG. 7 is a plan view (A) of a schematic block diagram illustrating a decontamination system according to a third embodiment of the present invention;
FIG. 8 is a front view (B) of the schematic block diagram illustrating the decontamination system according to the third embodiment of the present invention;
FIG. 9 is a side view (C) of the schematic block diagram illustrating the decontamination system according to the third embodiment of the present invention;
FIG. 10 is a schematic block diagram illustrating a decontamination system according to a fourth embodiment of the present invention;
FIG. 11 is a perspective view illustrating a first exemplary combination of a secondary mist generation device and a mist dispersion/diffusion device in the fourth embodiment;
FIG. 12 is a right side view illustrating the secondary mist generation device in FIG. 11 seen from the side of the room to be decontaminated;
FIG. 13 is a cross-sectional view illustrating the secondary mist generation device in FIG. 11 seen from the front in the room to be decontaminated;
FIG. 14 is a perspective view illustrating a second exemplary combination of a secondary mist generation device and a mist dispersion/diffusion device in the fourth embodiment;
FIG. 15 is a perspective view illustrating a third exemplary combination of a secondary mist generation device and a mist dispersion/diffusion device in the fourth embodiment;
FIG. 16 is a perspective view illustrating a fourth exemplary combination of a secondary mist generation device and a mist dispersion/diffusion device in the fourth embodiment;

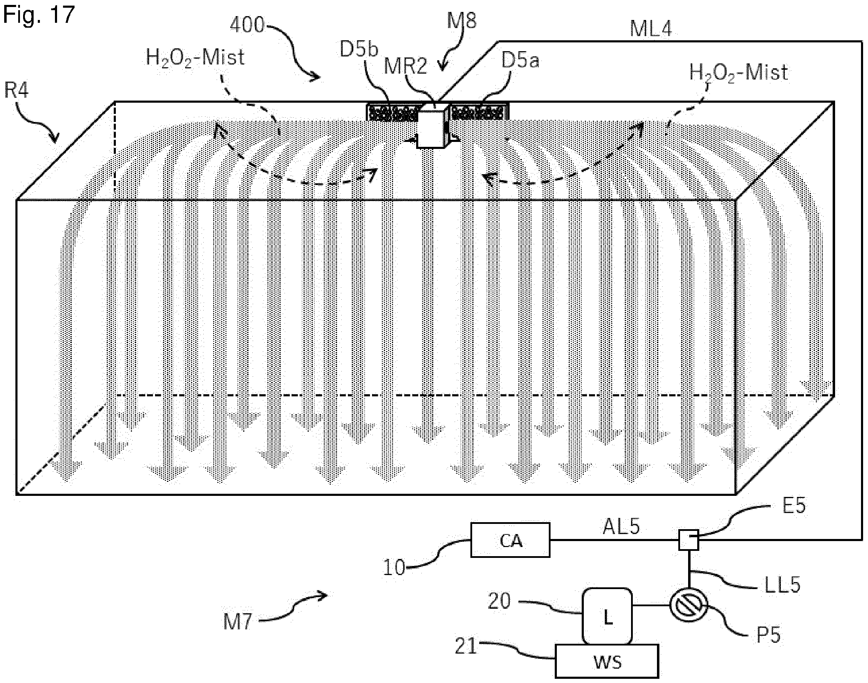

FIG. 17 is an image diagram illustrating the state of a hydrogen peroxide mist dispersed and diffused in the decontamination system in FIG. 10; and
FIG. 18 is a schematic block diagram illustrating a decontamination system according to a fifth embodiment of the present invention.

### DESCRIPTION OF EMBODIMENTS

In the present invention, "mist" is broadly interpreted as the state of a liquid droplet of a decontamination agent refined and floating in the air, the state of a gas and a liquid agent of a decontamination agent in mixture, the state of the decontamination agent to repeat the change in phase between condensation and evaporation of a gas and a droplet, and the like. In terms of particle size as well, the mist is also broadly interpreted to include mists, fogs, and liquid droplets, which can be subclassified.

Accordingly, the mist according to the present invention is categorized into a "mist" (the size may be defined as 10 µm or less) or a "fog" (the size may be defined as 5 µm or less), and a mist having a larger particle size. In the present invention, ultrasonic vibration converts even a mist, a fog and a liquid droplet sized 3 to 10 µm or more into equalized ultrafine particles 3 µm or less to provide high-level decontamination effects.

The decontamination system according to the present invention will be described with reference to each of the embodiments. The present invention is not restricted to each of the following embodiments.

### <First embodiment>

This first embodiment describes the case where a decontamination device of the present invention is disposed for one room to be decontaminated. This first embodiment additionally describes the case where a primary mist generation means and a secondary mist generation means are used in combination as a hydrogen peroxide mist generation means to discharge a fine, atomized secondary mist into the inside of a room to be decontaminated as a mist for decontamination.

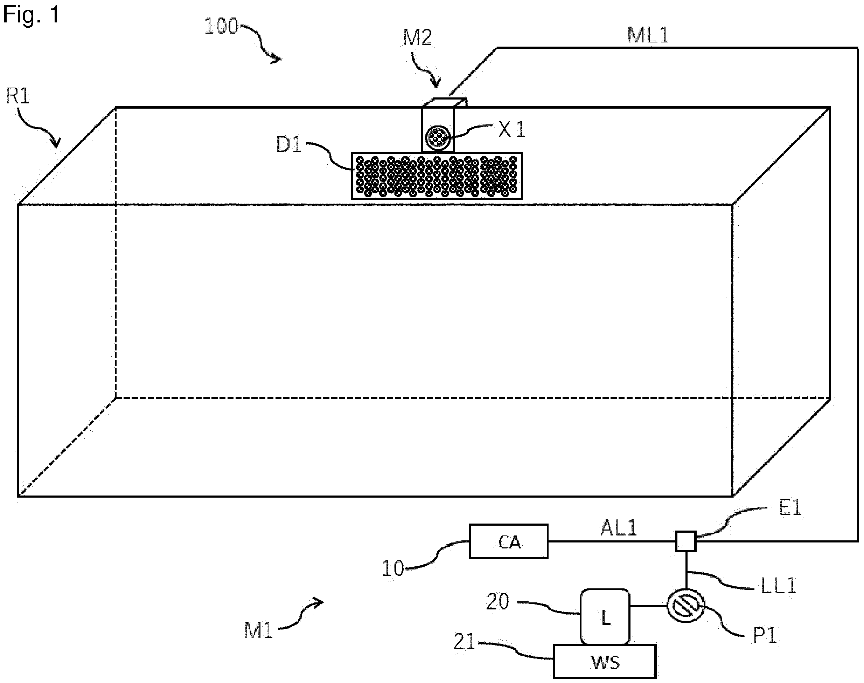

FIG. 1 is a schematic block diagram illustrating a decontamination system according to a first embodiment of the present invention. In FIG. 1, a set of hydrogen peroxide mist generation devices (in this first embodiment, a combination of a primary mist generation device and a secondary mist generation device) discharge a mist for decontamination into one isolator (volume: 4m³) as a room to be decontaminated. Also, description of a device inside the isolator, for example, a circulating fan, a HEPA filter, a straightening plate and so on is omitted, and only a space to be decontaminated is described. In this first embodiment, an isolator is defined as a room to be decontaminated, but the isolator is not restricted thereto, and may be a RABS, a clean room, a passing room, a pass box, or a combination thereof. Also, there may be one or more rooms to be decontaminated.

In FIG. 1, a decontamination system 100 disposed in a room to be decontaminated R1 includes a primary mist generation device M1, a secondary mist generation device M2, a mist discharge port X1, and a mist dispersion/diffusion device D1.

The primary mist generation device M1 includes an air compressor 10, a hydrogen peroxide solution tank 20, and an ejector E1 generating a primary mist.

The air compressor 10 acts as a compressed air generation means for generating compressed air, which is used as a carrier gas for conveying a hydrogen peroxide solution. In this first embodiment, the air compressor 10 is disposed so as to be separate from the room to be decontaminated R1.

The hydrogen peroxide solution tank 20 acts as a decontamination liquid supply means for storing a hydrogen peroxide solution, which is the source of a hydrogen peroxide mist as a mist for decontamination. In this first embodiment, the hydrogen peroxide solution tank 20 is disposed adjacent to the air compressor 10 so as to be separate from the room to be decontaminated R1. Herein, the concentration of the hydrogen peroxide solution stored in the hydrogen peroxide solution tank 20 is not particularly restricted, and in general, it is preferably 30 to 35 % by weight in view of hazardous materials in use. Also, the hydrogen peroxide solution tank 20 includes a weighing device 21 detecting the remaining amount of a hydrogen peroxide solution therein and a controller (not shown) controlling the remaining amount.

The ejector E1 generates a primary mist by mixing a hydrogen peroxide solution with compressed air. The ejector E1 is disposed adjacent to the air compressor 10 and the hydrogen peroxide solution tank 20 so as to be separate from the room to be decontaminated R1.

In FIG. 1, the decontamination system 100 includes an air supply pipe AL1 communicating the air compressor 10 and the ejector E1, a decontamination liquid supply pipe LL1 communicating the hydrogen peroxide solution tank 20 and the ejector E1, and a primary mist supply pipe ML1 communicating the ejector E1 and the secondary mist generation device M2.

The air supply pipe AL1 communicates a discharge port of the air compressor 10 and a driving flow path (not shown) of the ejector E1. A conduit line of the air supply pipe AL1 is provided with an on-off valve (not shown) controlling the supply of compressed air. Herein, the material and diameter of the air supply pipe AL1 are not particularly restricted, and in general, such a pipe is preferably a stainless pipe having an internal diameter of 1 to 10 mm. A conduit line between the air compressor 10 and the air supply pipe AL1 may be provided with an air dryer, an air regulator, an auto-drain, an oil mist separator, and other filter (each not shown in FIG. 1).

The decontamination liquid supply pipe LL1 communicates a supply port of the hydrogen peroxide solution tank 20 and a suction flow path (not shown) of the ejector E1. A conduit line of the decontamination liquid supply pipe LL1 is provided with a tube pump P1 controlling the supply of a hydrogen peroxide solution. Herein, the material and diameter of the decontamination liquid supply pipe LL1 are not particularly restricted so long as they can serve for a hydrogen peroxide solution, and in general, such a pipe is preferably a stainless pipe having an internal diameter of 1 to 10 mm.

The primary mist supply pipe ML1 communicates a discharge flow path of the ejector E1 and a secondary mist generation device M2 including an ultrasonic atomizer (later-described). The primary mist supply pipe ML1 is installed over a long distance from the vicinity of the air compressor 10 and the hydrogen peroxide solution tank 20 to the secondary mist generation device M2 disposed in the center of an upper portion on a corner wall surface of the room to be decontaminated R1. Herein, the material and diameter of the primary mist supply pipe ML1 may preferably be determined so long as a required amount of hydrogen peroxide mist can be conveyed over a long distance per unit time, and in general, such a pipe is preferably a stainless pipe having an internal diameter of 1 to 10 mm.

As illustrated in FIG. 1, the conveyance distance of the primary mist supply pipe ML1 is longer than the conveyance distance of the air supply pipe AL1 or the conveyance distance of the decontamination liquid supply pipe LL1. The conveyance distance of the primary mist by the primary mist supply pipe ML1 is not particularly restricted, and normally is 3 to 100 m or so. Meanwhile, the conveyance distance of the air supply pipe AL1 or the conveyance distance of the decontamination liquid supply pipe LL1 can be shortened.

In this first embodiment, the primary mist is a high-density mixing gas/liquid of compressed air and hydrogen peroxide solution with a high conveyance speed, and the primary mist supply pipe ML1 used may be a small-diameter pipe. Therefore, the room to be decontaminated R1 can be provided with a long primary mist supply pipe ML1. Accordingly, large-scale equipment such as large-diameter ducts is not required.

Also, a hydrogen peroxide solution in a primary mist is present as a liquid, thereby requiring no warming of a primary mist supply pipe ML1 to prevent condensation. Therefore, even in cases where a long pipe is installed for a room to be decontaminated R1, large-scale equipment such as anti-condensation heaters is not required.

Thus, the shorter conveyance distance of the decontamination liquid supply pipe LL1 can accurately determine the amount of a hydrogen peroxide solution supplied to the ejector E1. Accordingly, the amount of the hydrogen peroxide solution supplied to the secondary mist generation device M2 can accurately be determined, and the amount of a hydrogen peroxide mist discharged into the room to be decontaminated R1 is clearly determined. Meanwhile, the hydrogen peroxide solution in the primary mist, which is present as a liquid, is not condensed, thereby conveying a hydrogen peroxide solution over a long distance and accurately by elongating the conveyance distance of the primary mist supply pipe ML1. Moreover, complete conveyance of the hydrogen peroxide solution in the primary mist supply pipe ML1 by compressed air allows no residual dead liquid to stay in the pipe.

The secondary mist generation device M2 is disposed in the center of the upper portion (outer wall side) on the corner wall surface of the room to be decontaminated R1 to discharge a secondary mist into the inside of room to be decontaminated R1 through the mist discharge port X1 that is opened on the inner wall side of the room to be decontaminated R1. The secondary mist generation device M2 is composed of a mist receiving container and an ultrasonic atomizer and receives a primary mist containing a hydrogen peroxide solution conveyed from the ejector E1 to convert the same into a secondary mist. The structure and function of the mist receiving container and the ultrasonic atomizer will be described later.

The mist dispersion/diffusion device D1 is disposed laterally and lengthwise along the corner wall surface of the room to be decontaminated R1 to be symmetrical with respect to the mist discharge port X1 that is opened in the center an upper portion of the corner wall surface and to be beneath the mist discharge port X1 to disperse and diffuse a hydrogen peroxide mist (secondary mist) discharged from the secondary mist generation device M2 into the inside of the room to be decontaminated R1 uniformly to the inside of the room to be decontaminated R1. The structure and function of the mist dispersion/diffusion device D1 will be described later.

Thus, the secondary mist generation device M2 and the mist dispersion/diffusion device D1 are preferably disposed at the upper portion of the room to be decontaminated R1. The hydrogen peroxide mist can uniformly be dispersed and diffused downward by allowing itself, which is not only a fine mist, but also a liquid droplet, to fall under its own weight.

The position of the mist dispersion/diffusion device D1 is not restricted to that beneath the mist discharge port X1, and it may be any position so long as it is disposed adjacent to a lower portion of the mist discharge port X1. The distance from the mist discharge port X1 is not particularly restricted, and it may be, for example, 0 to 800 mm, preferably 0 to 300 mm. By allowing the mist dispersion/diffusion device D1 to be located adjacent to (incl. beneath) the lower portion of the mist discharge port X1, the hydrogen peroxide mist that is discharged into the inside of the room to be decontaminated R1 and tends to fall downward by gravity can more efficiently be dispersed and diffused.

In this first embodiment, the secondary mist generation device M2 and the mist dispersion/diffusion device D1 are disposed in the center of the upper portion of the corner wall surface of the room to be decontaminated R1. In addition, the positions of the secondary mist generation device M2 and the mist dispersion/diffusion device D1 are not restricted thereto, and they may be disposed on other wall surface. Even in this case, the secondary mist generation device M2 and the mist dispersion/diffusion device D1 are preferably disposed at positions where the hydrogen peroxide mist can uniformly be dispersed and diffused into the inside of the room to be decontaminated R1. Other arrangement will be described in the following third embodiment.

Large-capacity rooms to be decontaminated are provided with a plurality of hydrogen peroxide mist generation devices and a plurality of mist dispersion/diffusion devices associated therewith. Accordingly, a hydrogen peroxide mist can uniformly be dispersed and diffused into the inside of a room to be decontaminated to improve the decontamination efficiency.

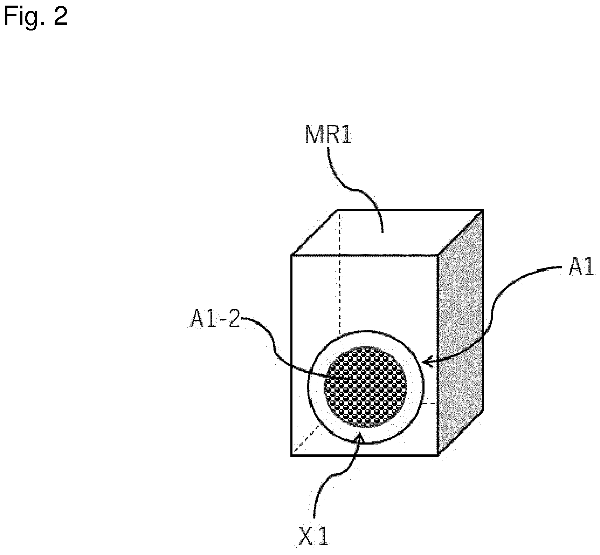

Subsequently, the secondary mist generation device M2 will be described. FIG. 2 is an external perspective view of a secondary mist generation device used in the first embodiment. In FIG. 2, the secondary mist generation device M2 is composed of a mist receiving container MR1 and an ultrasonic atomizer A1 to generate a secondary mist. The mist receiving container MR1 receives a primary mist containing a hydrogen peroxide solution conveyed from the ejector E1 and subjects the same to gas-liquid separation. Also, the mist receiving container MR1 supplies the separated hydrogen peroxide solution to the ultrasonic atomizer A1 and discharges the separated air into the outside. The mist discharge port X1 is provided in front of the mist receiving container MR1.

The ultrasonic atomizer A1 receives the hydrogen peroxide solution subjected to gas-liquid separation from the mist receiving container MR1, generates a fine hydrogen peroxide mist (secondary mist), and discharges the same into the inside of the room to be decontaminated R1. In this first embodiment, a vibration plate (later-described) of the ultrasonic atomizer A1 also serves as the mist discharge port X1.

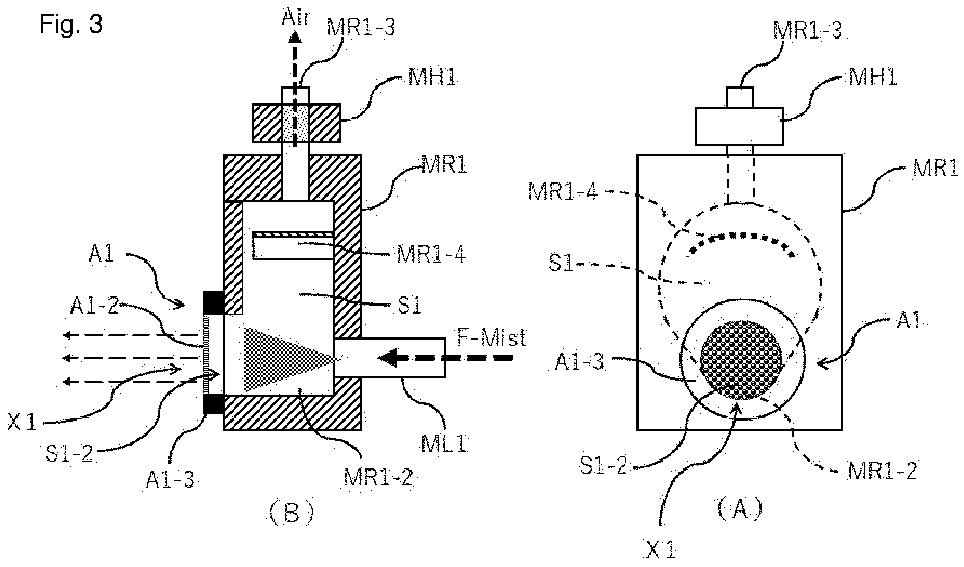

Herein, one exemplary secondary mist generation device M2 used in this first embodiment will be described. FIG. 3 illustrates the structure of the secondary mist generation device. FIG. 3(A) is a front view of the secondary mist generation device seen from a room to be decontaminated side, and FIG. 3(B) is a cross-sectional of the secondary mist generation device seen from the right side.

The mist receiving container MR1 constitutes an internal space S1 with a front internal portion having a semi-spindle-shaped cross section, and an ultrasonic atomizer A1 is attached to an opening S1-2 that is opened on an outer wall surface corresponding to a front lower end portion having a semi-spindle-shaped focusing width. The lower end portion of the internal space S1 is provided with a focusing width to serve as a liquid pool MR1-2 for a small amount of decontamination liquid subjected to gas-liquid separation. Also, an end of the primary mist supply pipe ML1 communicates with a rear lower end portion of the mist receiving container MR1 (at a position opposite the ultrasonic atomizer A1) toward the inside of the mist receiving container MR1. An air vent MR1-3 is opened at an upper end portion inside a rear surface of the mist receiving container MR1. Also, a path of the air vent MR1-3 may be provided with a filter MH1 resolving hydrogen peroxide. In addition, a baffle plate MR1-4 is provided between an end of the primary mist supply pipe ML1 in the center inside the mist receiving container MR1 and the air vent MR1-3.

The ultrasonic atomizer A1 is composed of a substantially annular disk-shaped perforated vibration plate A1-2 provided with a plurality of micropores (not shown) atomizing the decontamination liquid (hydrogen peroxide solution) subjected to gas-liquid separation, the micropores passing through the perforated vibration plate between the front surface and the back surface thereof, a piezoelectric vibrator A1-3 formed of a substantially annular disk in which the perforated vibration plate A1-2 is subjected to film vibration, and a controller (not shown) controlling the vibration of the piezoelectric vibrator A1-3. The perforated vibration plate A1-2 is affixed to the piezoelectric vibrator A1-3 so as to cover an internal hole portion of the piezoelectric vibrator A1-3.

Also, the perforated vibration plate A1 is attached such that the front surface faces the inside of the room to be decontaminated and the rear surface faces the inside of the mist receiving container MR1, and a plurality of micropores of the perforated vibration plate A1-2 passes through the inside of the room to be decontaminated and the inside of the mist receiving container MR1. In this first embodiment, the front surface of the perforated vibration plate A1-2 also serves as the mist discharge port X1. In FIG. 3, the perforated vibration plate A1-2 is disposed so as to discharge a hydrogen peroxide mist horizontally from the front surface of the perforated vibration plate A1-2, but the configuration is not restricted thereto, and the hydrogen peroxide mist may be discharged downward or upward, depending on the position of the plate disposed.

In this state, the primary mist is discharged into the inside of the mist receiving container MR1 through the primary mist supply pipe ML1. The rear surface of the perforated vibration plate A1-2 and an end of the primary mist supply pipe ML1 are opposite each other inside the mist receiving container MR1. Accordingly, the discharged primary mist is ejected directly onto the rear surface of the perforated vibration plate A1-2 to be subjected to gas-liquid separation. The separated decontamination liquid is converted into a fine secondary mist (hydrogen peroxide mist) through a plurality of micropores of the perforated vibration plate A1-2 under ultrasonic vibration to be discharged into the inside of the room to be decontaminated and to provide decontamination effects. Even if part of the decontamination liquid subjected to gas-liquid separation on the rear surface of the perforated vibration plate A1-2 is retained in the liquid pool MR1-2, this is converted into a fine secondary mist, though in very small quantities, through a plurality of micropores of the perforated vibration plate A1-2 to be discharged into the inside of the room to be decontaminated. Meanwhile, the separated air is discharged from the air vent MR1-3 into the outside.

Thus, since the amount of a decontamination liquid supplied to the ultrasonic atomizer A1 can accurately be controlled to the least possible amount, presence of residual decontamination liquid can be avoided for efficient decontamination even in cases where long pipes are installed for each room of a plurality of rooms to be decontaminated. Moreover, since a precise amount of hydrogen peroxide mist can be thus supplied for each room, no shortage of decontamination liquid can occur and no failure is found on an ultrasonic vibrator, which is the core of the ultrasonic atomizer A1. In addition, sufficient decontamination effects can be provided with the least possible amount of decontamination liquid to efficiently utilize a decontamination liquid.

Herein, the diameter and number of micropores of the perforated vibration plate A1-2 are not particularly restricted, and they may be determined so long as ultrasonic atomization effects and sufficient amount of a hydrogen peroxide mist can be provided. The diameter is normally 4 to 11 µm, but if it is less than a bacterial spore (e.g., 0.5 to about 3 µm or so), filtering effects are provided to cause no bacterial decontamination on a decontamination liquid.

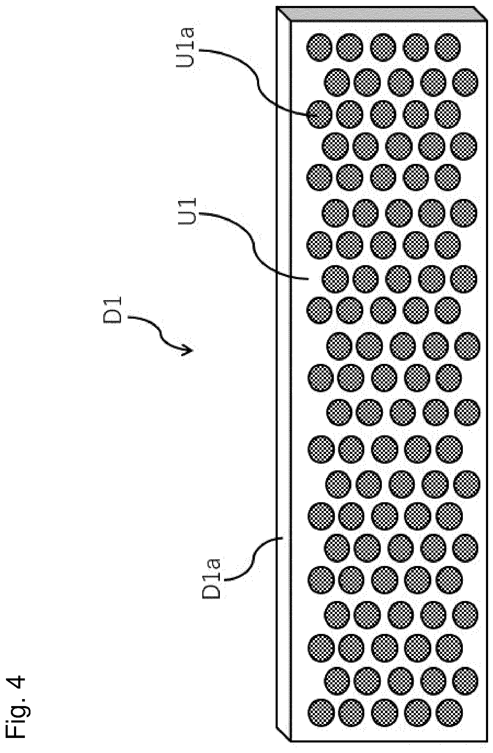

Subsequently, a mist dispersion/diffusion device D1 for dispersing and diffusing the hydrogen peroxide mist thus discharged into the inside of a room to be decontaminated will be described. FIG. 4 is an external perspective view of a mist dispersion/diffusion device used in the first embodiment. In FIG. 4, the mist dispersion/diffusion device D1 includes an ultrasonic vibration plate D1a. The ultrasonic vibration plate D1a is disposed such that acoustic radiation pressure by ultrasonic vibration from the rear of a lower portion in the discharge direction acts on the hydrogen peroxide mist discharged from the ultrasonic atomizer A1 (see FIG. 1).

Herein, the structure and operation of the ultrasonic vibration plate D1a will be described. In FIG. 4, the ultrasonic vibration plate D1a includes a base and a plurality of transmitters. In this first embodiment, a base U1 is used, and the transmitter used is an ultrasonic transmitter U1a. In this first embodiment, 105 ultrasonic transmitters U1a are arranged on the base U1 so as to be uniform in transmission direction of a vibrating surface thereof (slightly leftward as seen from the front shown). The number of ultrasonic transmitters is not particularly restricted.

In this first embodiment, an ultra directional ultrasonic transmitter U1a is used. Specifically, an ultrasonic transmitter (DC12V, 50mA) of frequency modulation system for transmitting an ultrasound whose frequency is around 40 KHz is used. The type, size, structure and output of the ultrasonic transmitter are not particularly restricted. In the present invention, the ultrasonic vibration plate is not restricted to an ultrasonic transmitter, and the ultrasonic generation mechanism, frequency range and output are not particularly restricted.

In this first embodiment, a plurality of (105) ultrasonic transmitters U1a are arranged so as to be uniform in transmission direction of the vibrating surface, and the transmitters are operated in the same phase to mutually amplify ultrasounds from the plurality of ultrasonic transmitters U1a in the front direction and mutually cancel out ultrasounds from the plurality of ultrasonic transmitters U1a in the lateral direction. Consequently, the ultrasonic transmitters U1a arranged on the base U1 are subjected to ultrasonic vibration to generate a significantly directional sound flow traveling in the air from each of the vibrating surfaces in the vertical direction. In this first embodiment, a controller (not shown) controls the frequency, output, and transmission time of an ultrasonic transmitter U1a, and the pressure on the hydrogen peroxide mist by acoustic radiation pressure can essentially be changed in intermittent operation or stronger/weaker operation of ultrasonic transmission (later-described).

Subsequently, a method for decontaminating a room to be decontaminated R1, using a decontamination system 100 of this first embodiment, will be described. Herein, the secondary mist generation device M2 described in FIGS. 2 and 3 and the mist dispersion/diffusion device D1 described in FIG. 4 are disposed in the center of the upper portion on the corner wall surface of the room to be decontaminated R1 (see FIG. 1).

In this first embodiment, the amount of a hydrogen peroxide mist to be discharged per unit time is determined for the room to be decontaminated R1. The amount of a hydrogen peroxide solution, which is supplied from the hydrogen peroxide solution tank 20 through the decontamination liquid supply pipe LL1 for the ejector E1 corresponding to the room to be decontaminated R1, is determined from the amount of the mist for decontamination discharged. In addition, predetermined conditions are preferably set for the room to be decontaminated R1 before decontamination, using temperature regulation equipment and humidity regulation equipment.

Subsequently, a decontamination operation will be started. First, an on-off valve (not shown) of the air supply pipe AL1 is opened to supply compressed air from the air compressor 10 to a driving flow path of the ejector E1 through the air supply pipe AL1. Herein, the compressed air supplied to the ejector E1 is not particularly restricted, and the discharge pressure is preferably 0.05 MPa or more, and the air flow amount is preferably 0.5 to 20 NL/min. The air flow amount may be determined according to the concentration and amount of a hydrogen peroxide solution supplied to the room to be decontaminated R1 and the distance to the room to be decontaminated R1.

Subsequently, a tube pump P1 of the decontamination liquid supply pipe LL1 is operated to supply a hydrogen peroxide solution from the hydrogen peroxide solution tank 20 to a suction flow path of the ejector E1 through the decontamination liquid supply pipe LL1. Also, the amount of the hydrogen peroxide solution supplied corresponds to that determined as above for the ejector E1. Herein, the concentration of the hydrogen peroxide solution supplied to the ejector E1 is not particularly restricted, and in general, it may be 30 to 35 % by weight as currently used, or may be used by concentrating or diluting the solution. The flow amount of the hydrogen peroxide solutions supplied to the ejector E1 may be 0.5 to 10 g/min.

With the amounts of a hydrogen peroxide solution and compressed air being in the above ranges, a primary mist obtained by mixing a hydrogen peroxide solution through the primary mist supply pipe ML1 can be conveyed even over a long distance.

By the above operation, the ejector E1 converts the hydrogen peroxide solution and compressed air into a primary mist, which is supplied to the mist receiving container MR1 that constitutes the secondary mist generation device M2 through the primary mist supply pipe ML1 from the discharge flow path of the ejector E1.

In the mist receiving containers MR1, the primary mist is subjected to gas-liquid separation to be separated into a hydrogen peroxide solution and air. The hydrogen peroxide solution subjected to gas-liquid separation in the mist receiving container MR1 is supplied to the ultrasonic atomizer A1 inside the room to be decontaminated R1 from the mist receiving container MR1. As described above, the perforated vibration plate A1-2 of the ultrasonic atomizer A1 also serves as the mist discharge port X1. At this stage, a piezoelectric vibrator A1-3 of the ultrasonic atomizer A1 starts operation. Accordingly, a fine hydrogen peroxide mist generated in the ultrasonic atomizer A1 is discharged into the inside of the room to be decontaminated R1. At this time, the hydrogen peroxide mist is discharged horizontally perpendicular to the corner wall surface from the mist discharge port X1 that is opened in the center of the upper portion of the corner wall surface of the room to be decontaminated R1.

Herein, a controller (not shown) included in the mist dispersion/diffusion device D1 changes the operation of the ultrasonic vibration plate D1a according to a predetermined program. Specifically, the hydrogen peroxide mist is subjected to intermittent operation by turning on/off the output of acoustic radiation pressure acting from the rear of a lower portion or to stronger/weaker operation by turning the output stronger/weaker. At the same time, the transmission time and suspension time are adjusted. Accordingly, the hydrogen peroxide mist discharged horizontally perpendicular to the corner wall surface from the center of the upper portion of the corner wall surface of the room to be decontaminated R1 is pressed by acoustic radiation pressure in variation mode. Accordingly, the directions of dispersing and diffusing a hydrogen peroxide mist change according to a predetermined program.

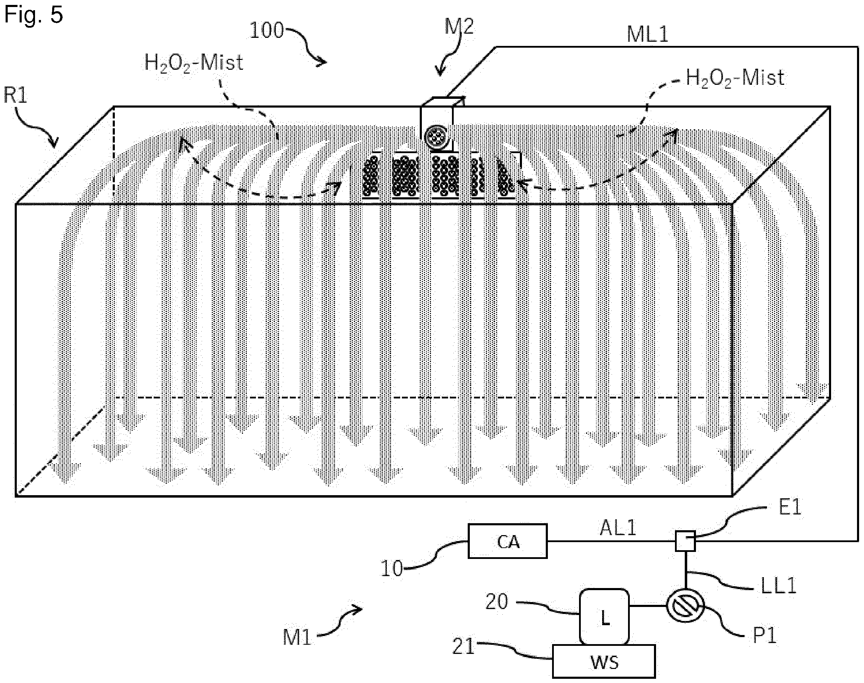

FIG. 5 is an image diagram illustrating the state of a hydrogen peroxide mist dispersed and diffused in a decontamination system of this first embodiment. In FIG. 5, the hydrogen peroxide mist (HO₂O₂-Mist) discharged from the ultrasonic atomizer A1 of the secondary mist generation device M2 is first uniformly dispersed and diffused horizontally in the room to be decontaminated R1 according to changing operations of the ultrasonic vibration plate D1a of the mist dispersion/diffusion device D1. Also, the hydrogen peroxide solution mist is further refined by acoustic radiation pressure to be dispersed and diffused into the inside of the room to be decontaminated R1.

Subsequently, the hydrogen peroxide mist uniformly dispersed and diffused horizontally in the room to be decontaminated R1 by changing operations of the ultrasonic vibration plate D1a is composed of extremely fine liquid droplets, which slowly fall under its own weight. Therefore, the hydrogen peroxide mist is uniformly dispersed and diffused in the vertical direction in the room to be decontaminated R1 as well.

In this first embodiment, acoustic radiation pressure by ultrasonic vibration further refines the hydrogen peroxide solution mist and converts even a mist, a fog and a liquid droplet sized 3 to 10 µm or more into equalized ultrafine particles 3 µm or less to provide high-level decontamination effects. In fact, since the hydrogen peroxide mist is refined by ultrasonic vibration to have smaller particle sizes and larger surface areas, it is believed that the evaporation efficiency of mists is high, resulting in repeated evaporation and condensation. The hydrogen peroxide mist is a highly-refined mist to form a uniform and thin condensed film on an internal wall surface of the room to be decontaminated R1. Accordingly, it is believed that ultrafine particles of hydrogen peroxide 3µm or less and a hydrogen peroxide gas are subjected to phase change for coexistence inside the room to be decontaminated R1 to provide a high-level decontamination environment.

Accordingly, a hydrogen peroxide mist is discharged for a predetermined period of time to provide acoustic radiation pressure. After the predetermined period of time has elapsed, the tube pump P1 of the decontamination liquid supply pipes LL1 is stopped to stop supply of a hydrogen peroxide solution. At this stage, compressed air is being supplied to the ejector E1 through the air supply pipe AL1, and a residual hydrogen peroxide solution in the primary mist supply pipe ML1 is all sent to the mist receiving container MR1. Accordingly, a predetermined amount of hydrogen peroxide mist is accurately discharged into the room to be decontaminated R1. When the hydrogen peroxide solution supplied to the ultrasonic atomizer A1 is all converted into a mist, the ultrasonic atomizer A1 and the mist dispersion/diffusion device D1 stop operation.

Subsequently, an on-off valve of the air supply pipe AL1 is closed to stop supply of compressed air. Thereafter, the hydrogen peroxide mist inside the room is discharged to aerate the inside of the room and complete the decontamination operation. Each of the above operations is preferably automatically controlled using a micro-computer.

Accordingly, by repeated re-evaporation and condensation of the uniformly and thinly formed condensed film on the internal wall surface of the room to be decontaminated R1, the concentration of a decontamination agent in a decontamination mist can be increased and efficient decontamination can be performed with a small amount of decontamination agent. Such an efficient decontamination and condensation prevention with a small amount of decontamination agent can improve the efficiency of aeration after decontamination and reduce the duration of decontamination operations.

Herein, in this first embodiment, the state of ultrafine hydrogen peroxide mists to be uniformly dispersed and diffused horizontally and vertically was confirmed. Specifically, one secondary mist generation device M2, and one mist dispersion/diffusion device D1 are disposed at positions illustrated in FIG. 1 for an isolator (Volume: W3×1D×1.3H=4m³), which is the room to be decontaminated R1 of this first embodiment. A planned standard decontamination capacity was 15 g/m³, and the amount of the hydrogen peroxide solution (35 % by weight) discharged from a set of hydrogen peroxide mist generation devices (in this first embodiment, a combination of a primary mist generation device M1 and a secondary mist generation device M2) into the inside of the room to be decontaminated R1 was 2 g/min and the solution was fed for 30 minutes.

A plurality of condensation sensors SDM (developed by inventors of the present invention; see JP-A-3809176 for details) are disposed at main locations on a bottom wall surface of an isolator, and the thickness of a condensed film was measured to confirm the SDV value (value for confirming the formation of condensed films best suited for decontamination). Consequently, it was confirmed that a hydrogen peroxide mist is sufficiently dispersed and diffused horizontally and vertically by operating a secondary mist generation device M2 and a mist dispersion/diffusion device D1. Meanwhile, according to a Comparative Example, the only operation of discharging a hydrogen peroxide mist from a secondary mist generation device M2 after stopping the operation of the mist dispersion/diffusion device D1 leads to insufficient horizontal dispersion and diffusion and fails to decontaminate the isolator favorably at several locations. Condensation was partially found on the bottom wall surface in the discharge direction from the secondary mist generation device M2.

### <Second embodiment>

This second embodiment describes the case where a decontamination device of the present invention is disposed for one room to be decontaminated as in the above first embodiment. This second embodiment additionally describes the case where a single mist generation means is used as a hydrogen peroxide mist generation means to discharge a fine mist into the inside of a room to be decontaminated as a mist for decontamination.

In this second embodiment, without using a secondary mist generation device M2 (incl. ultrasonic atomizer A1) used in the above first embodiment, a hydrogen peroxide solution is directly converted into a mist to be discharged into the inside of the room to be decontaminated. Therefore, the particle size of generated mists is preferably made small. The mist generation device may be of any structure so long as it can convert a liquid hydrogen peroxide solution into a fine mist.

The mist generation device may be, for example, a single-fluid spray nozzle directly converting a liquid into a mist, a piezo-high pressure injection device, a dipping-type ultrasonic atomizer, a disk-type atomizer, and a disk-mesh-type atomizer. In addition, the mist generation device may be an ejector converting a liquid into a mist with high-pressure air or the like or a two-fluid spray nozzle. This second embodiment describes the case where an ejector converting a decontamination agent into a mist with high-pressure air is employed.

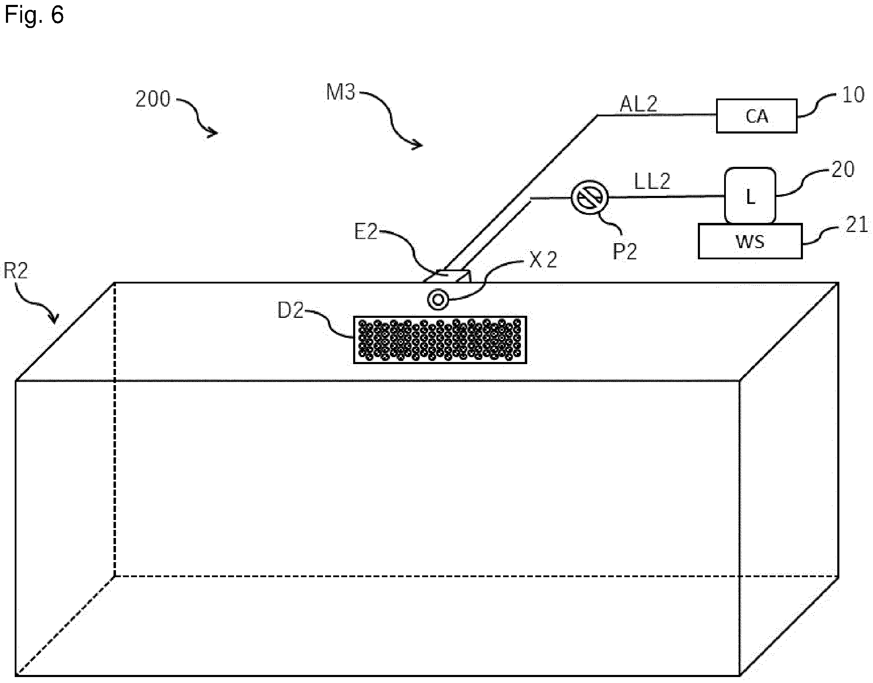

FIG. 6 is a schematic block diagram illustrating a decontamination system according to a second embodiment of the present invention. In FIG. 2, as in the above first embodiment, a hydrogen peroxide mist generation device (in this embodiment, only a single mist generation device is used) discharges a mist for decontamination into one isolator (volume: 4m³) as a room to be decontaminated. Also, description of a device inside the isolator, for example, a circulating fan, a HEPA filter, a straightening plate and so on is omitted, and only a space to be decontaminated is described.

In FIG. 6, a decontamination system 200 disposed in a room to be decontaminated R2 includes a mist generation device M3, a mist discharge port X2, and a mist dispersion/diffusion device D2.

The mist generation device M3 includes an air compressor 10, a hydrogen peroxide solution tank 20, and an ejector E2 generating a mist.

The configuration and structure of the air compressor 10 and the hydrogen peroxide solution tank 20 are the same as in the above first embodiment, and its description is herein omitted. Unlike in the above first embodiment, the ejector E2 is disposed in the center of an upper portion (outer wall side) on a corner wall surface of the room to be decontaminated R2 to discharge a mist into the inside of room to be decontaminated R2 through the mist discharge port X2 that is opened on the inner wall side of the room to be decontaminated R2.

In FIG. 6, the decontamination system 200 includes an air supply pipe AL2 communicating the air compressor 10 and the ejector E2 and a decontamination liquid supply pipe LL2 communicating the hydrogen peroxide solution tank 20 and the ejector E2. The configuration and structure of the air supply pipe AL2 and the decontamination liquid supply pipe LL2 are the same as in the above first embodiment, and its description is herein omitted.

The mist dispersion/diffusion device D2 is disposed laterally and lengthwise along the corner wall surface of the room to be decontaminated R2 to be symmetrical with respect to the mist discharge port X2 that is opened in the center of an upper portion of the corner wall surface and to be beneath the mist discharge port X2 to disperse and diffuse a hydrogen peroxide mist discharged from the mist generation device M3 into the inside of the room to be decontaminated R2 uniformly to the inside of the room to be decontaminated R2. The configuration and structure of the mist dispersion/diffusion device D2 are the same as in the above first embodiment, and its description is herein omitted.

Thus, the mist generation device M3 and the mist dispersion/diffusion device D2 are preferably disposed at the upper portion of the room to be decontaminated R2. The hydrogen peroxide mist can uniformly be dispersed and diffused downward by allowing itself, which is not only a fine mist, but also a liquid droplet, to fall under its own weight.

The position of the mist dispersion/diffusion device D2 is not restricted to that beneath the mist discharge port X2, and it may be any position so long as it is disposed adjacent to a lower portion of the mist discharge port X2. The distance from the mist discharge port X2 is not particularly restricted, and it may be, for example, 0 to 800 mm, preferably 0 to 300 mm. By allowing the mist dispersion/diffusion device D2 to be located adjacent to (incl. beneath) the lower portion of the mist discharge port X2, the hydrogen peroxide mist that is discharged into the inside of the room to be decontaminated R2 and tends to fall downward by gravity can more efficiently be dispersed and diffused.

In this second embodiment, the mist generation device M3 and the mist dispersion/diffusion device D2 are disposed in the center of the upper portion of the corner wall surface of the room to be decontaminated R2. In addition, the positions of the mist generation device M3 and the mist dispersion/diffusion device D2 are not restricted thereto, and they may be disposed on other wall surface. Even in this case, the mist generation device M3 and the mist dispersion/diffusion device D2 are preferably disposed at positions where the hydrogen peroxide mist can uniformly be dispersed and diffused into the inside of the room to be decontaminated R2.

Large-capacity rooms to be decontaminated are provided with a plurality of hydrogen peroxide mist generation devices and a plurality of mist dispersion/diffusion devices associated therewith. Accordingly, a hydrogen peroxide mist can uniformly be dispersed and diffused into the inside of a room to be decontaminated to improve the decontamination efficiency.

Even in this second embodiment, as in the above first embodiment, an ultrasonic vibration plate of the mist dispersion/diffusion device D2 is disposed such that acoustic radiation pressure by ultrasonic vibration from the rear of a lower portion in the discharge direction acts on the hydrogen peroxide mist discharged from the ejector E2 (see FIG. 6). Therefore, a method for controlling a hydrogen peroxide mist in this second embodiment is the same as in the above first embodiment, and its description is herein omitted. Also, an image diagram illustrating the state of a hydrogen peroxide mist dispersed and diffused in this second embodiment is the same as in the above first embodiment (see FIG. 5).

### <Third embodiment>

This third embodiment describes the case where a decontamination device of the present invention is disposed for one room to be decontaminated having a large capacity. This third embodiment additionally describes the case where one primary mist generation means and 2 secondary mist generation means are used in combination as a hydrogen peroxide mist generation means to discharge a fine, atomized secondary mist into the inside of a room to be decontaminated from 2 locations as a mist for decontamination.

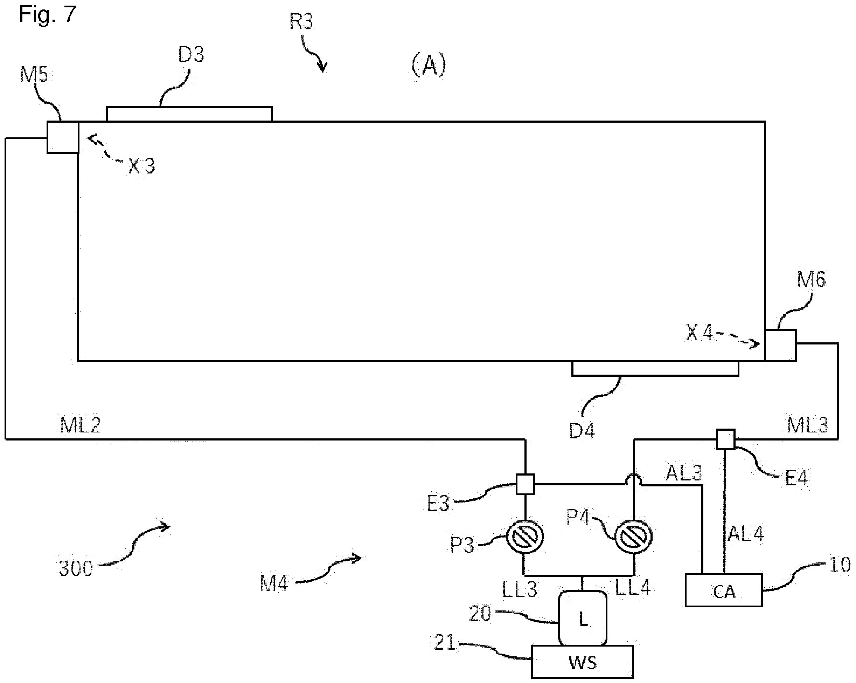

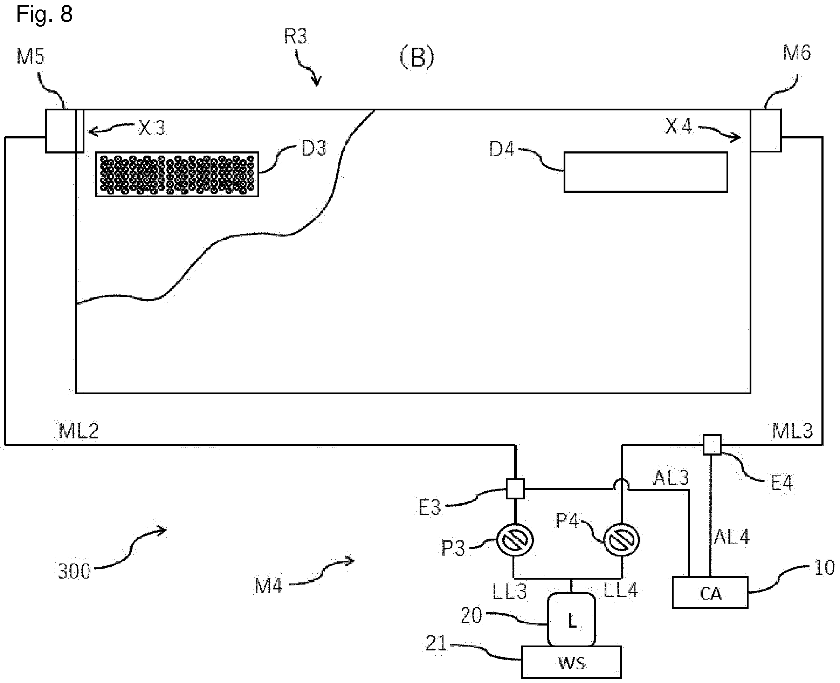

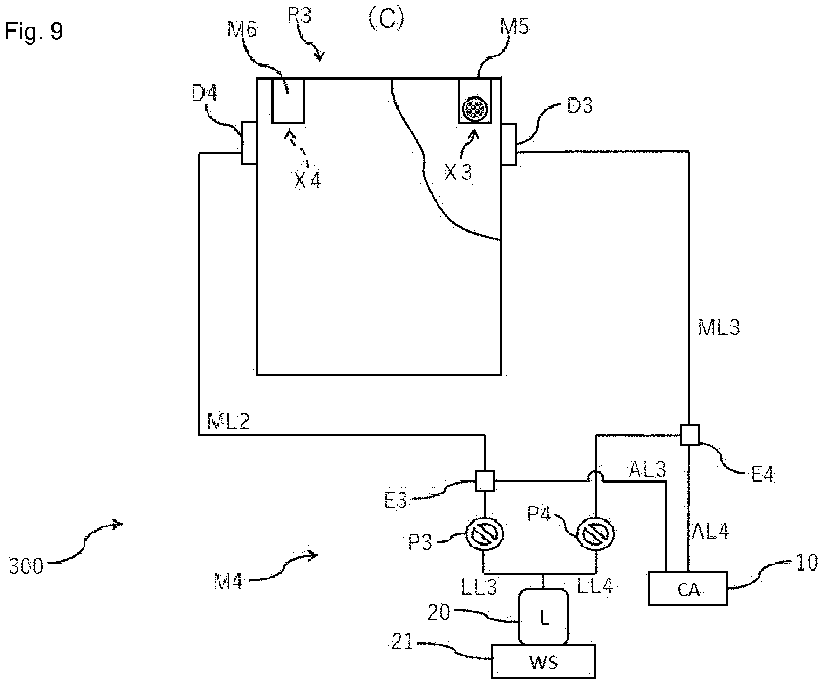

FIG. 7 is a plan view (A) of a schematic block diagram illustrating a decontamination system according to a third embodiment of the present invention. FIG. 8 is a front view (B) of the schematic block diagram illustrating the inside of the room to be decontaminated by partial break line. Likewise, FIG. 9 is a side view (C) of the schematic block diagram illustrating the inside of the room by partial break line. In FIGS. 7 to 9, a hydrogen peroxide mist generation device (in this third embodiment, a combination of one primary mist generation device and two secondary mist generation devices) discharges a mist for decontamination into one isolator (volume: 8m³) as a room to be decontaminated. Also, description of a device inside the isolator, for example, a circulating fan, a HEPA filter, a straightening plate and so on is omitted, and only a space to be decontaminated is described.

In FIGS. 7 to 9, a decontamination system 300 disposed in a room to be decontaminated R3 includes one primary mist generation device M4, 2 secondary mist generation devices M5, M6, 2 mist discharge ports X3, X4, and 2 mist dispersion/diffusion devices D3, D4.

The primary mist generation device M4 includes an air compressor 10, a hydrogen peroxide solution tank 20, and 2 ejectors E3, E4 generating a primary mist.

The configuration and structure of the air compressor 10, the hydrogen peroxide solution tank 20 and the 2 ejectors E3, E4 are the same as in the above first embodiment, and its description is herein omitted. The 2 ejectors E3, E4 supply a primary mist to the 2 secondary mist generation devices M5, M6, respectively. The 2 ejectors E3, E4 are disposed adjacent to the air compressor 10 and the hydrogen peroxide solution tank 20 so as to be separate from the room to be decontaminated R3.

In FIGS. 7 to 9, the decontamination system 300 includes 2 air supply pipes AL3, AL4 communicating the air compressor 10 and the 2 ejectors E3, E4, 2 decontamination liquid supply pipes LL3, LL4 communicating the hydrogen peroxide solution tank 20 and the 2 ejectors E3, E4, and 2 primary mist supply pipes ML2, ML3 communicating the 2 ejectors E3, E4 and the secondary mist generation devices M5, M6. The configuration and structure of the 2 air supply pipes AL3, AL4, the 2 decontamination liquid supply pipes LL3, LL4, and the 2 primary mist supply pipes ML2, ML3 are the same in the above first embodiment, and its description is herein omitted.

As shown in FIGS. 7 to 9, the conveyance distance totaling those of the 2 primary mist supply pipes ML2, ML3 is longer than the conveyance distance totaling those of the 2 air supply pipes AL3, AL4 or the conveyance distance totaling those of the 2 decontamination liquid supply pipes LL3, LL4. The conveyance distance for the primary mist by the 2 primary mist supply pipes ML2, ML3 is not particularly restricted, and normally is 3 to 100 m or so. Meanwhile, the conveyance distance totaling those of the 2 air supply pipes AL3, AL4 or the conveyance distance totaling those of the 2 decontamination liquid supply pipes LL3, LL4 can be shortened.

In this third embodiment, the primary mist is a high-density mixing gas/liquid of compressed air and hydrogen peroxide solution with a high conveyance speed, and 2 primary mist supply pipes ML2, ML3 used may each be a small-diameter pipe. Therefore, the room to be decontaminated R3 can be provided with long primary mist supply pipes ML2, ML3. Accordingly, large-scale equipment such as large-diameter ducts is not required.

Also, a hydrogen peroxide solution in a primary mist is present as a liquid, thereby requiring no warming of 2 primary mist supply pipes ML2, ML3 to prevent condensation. Therefore, even in cases where a long pipe is installed for the room to be decontaminated R3, large-scale equipment such as anti-condensation heaters is not required.

Thus, the shorter conveyance distance totaling those of the 2 decontamination liquid supply pipes LL3, LL4 can accurately determine the amount of a hydrogen peroxide solution supplied to the 2 ejectors E3, E4. Accordingly, the amount of the hydrogen peroxide solution supplied to the 2 secondary mist generation devices M5, M6 can accurately be determined, and the amount of a hydrogen peroxide mist discharged into the room to be decontaminated R3 is clearly determined. Meanwhile, the hydrogen peroxide solution in the primary mist, which is present as a liquid, is not condensed, thereby conveying a hydrogen peroxide solution over a long distance and accurately by elongating the conveyance distance totaling those of the 2 primary mist supply pipes ML2, ML3. Moreover, complete conveyance of the hydrogen peroxide solution in the 2 primary mist supply pipes ML2, ML3 by compressed air allows no residual dead liquid to stay in the pipe.

In this third embodiment, 2 mist dispersion/diffusion devices D3, D4 are disposed laterally and lengthwise on a wall surface adjacent to a lower side wall in the mist discharge direction of the mist discharge ports X3, X4 that are opened at diagonal positions at an upper corner position on a side wall surface of the room to be decontaminated R3 to disperse and diffuse a hydrogen peroxide mist (secondary mist) discharged from the primary mist generation device M4 into the inside of the room to be decontaminated R3 uniformly to the inside of the room to be decontaminated R3. The structure and function of the 2 mist dispersion/diffusion devices D3, D4 are the same as in the above first embodiment, and its description is herein omitted.

Even in this third embodiment, a controller (not shown) included in each of the 2 mist dispersion/diffusion devices D3, D4 changes the operation of each ultrasonic vibration plate according to a predetermined program. Specifically, the hydrogen peroxide mist is subjected to intermittent operation by turning on/off the output of acoustic radiation pressure acting from the side of a lower portion or to stronger/weaker operation by turning the output stronger/weaker. At the same time, the transmission time and suspension time are adjusted. Accordingly, the hydrogen peroxide mist discharged horizontally along a side wall surface from the upper corner portion of the side wall surface of the room to be decontaminated R3 is pressed by acoustic radiation pressure in variation mode. Accordingly, the directions of dispersing and diffusing a hydrogen peroxide mist change according to a predetermined program.

Thus, 2 secondary mist generation devices M5, M6 and 2 mist dispersion/diffusion devices D3, D4 are preferably disposed at diagonal positions of the upper portion of the room to be decontaminated R3. The hydrogen peroxide mist can uniformly be dispersed and diffused downward by allowing itself, which is not only a fine mist, but also a liquid droplet, to fall under its own weight.

The positions of the 2 mist dispersion/diffusion devices D3, D4 are not restricted so long as they are adjacent to the lower portion on the side wall surface in the mist discharge direction of the mist discharge ports X3, X4 corresponding to the devices D3, D4, respectively. Preferably, the lateral distance from each of the mist discharge ports X3, X4 (the distance in the discharge direction) is not so large. Meanwhile, the lower distance from each of the mist discharge ports X3, X4 is not particularly restricted, and it may be, for example, 0 to 800 mm, preferably 0 to 300 mm. By allowing the 2 mist dispersion/diffusion devices D3, D4 to be located adjacent to (incl. beneath) the lower portion of the mist discharge ports X3, X4 in the mist discharge direction, the hydrogen peroxide mist that is discharged into the inside of the room to be decontaminated R3 and tends to fall downward by gravity can more efficiently be dispersed and diffused.

### <Fourth embodiment>

This fourth embodiment describes the case where a decontamination device of the present invention is disposed for one room to be decontaminated as in the above first embodiment. In addition, in this fourth embodiment, as in the above first embodiment, a primary mist generation means and a secondary mist generation means are used in combination as a hydrogen peroxide mist generation means to discharge a fine, atomized secondary mist into the inside of a room to be decontaminated as a mist for decontamination. In this fourth embodiment, the arrangement of a hydrogen peroxide mist generation device and a mist dispersion/diffusion device is different from that in the first embodiment.

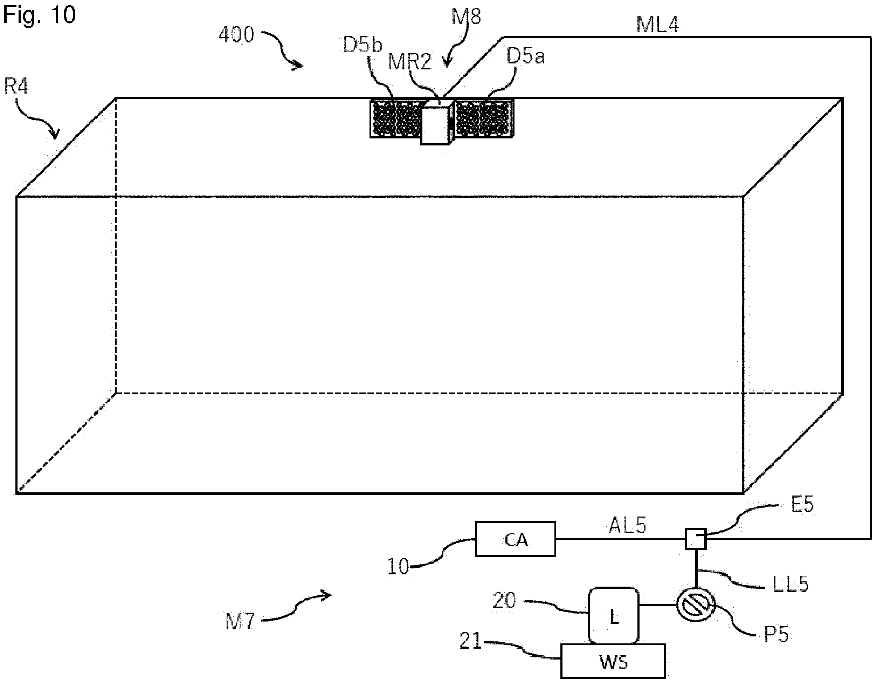

FIG. 10 is a schematic block diagram illustrating a decontamination system according to a fourth embodiment of the present invention. In FIG. 10, as in the above first embodiment, a set of hydrogen peroxide mist generation devices (in this fourth embodiment, a combination of a primary mist generation device and a secondary mist generation device) discharge a mist for decontamination into one isolator (volume: 4m³) as a room to be decontaminated. Also, description of a device inside the isolator, for example, a circulating fan, a HEPA filter, a straightening plate and so on is omitted, and only a space to be decontaminated is described.

In FIG. 10, a decontamination system 400 disposed in a room to be decontaminated R4 includes a primary mist generation device M7, a secondary mist generation device M8, and a mist dispersion/diffusion device D5. The primary mist generation device M7 includes an air compressor 10, a hydrogen peroxide solution tank 20, and an ejector E5 generating a primary mist. In FIG. 10, the room to be decontaminated R4 includes one primary mist generation device M7, one secondary mist generation device M8, and a set of 2 mist dispersion/diffusion devices D5a, D5b.

The configuration and structure of the air compressor 10, the hydrogen peroxide solution tank 20 and the ejector E5 are the same as in the above first embodiment, and its description is herein omitted. In FIG. 10, the decontamination system 400 includes an air supply pipe AL5 communicating the air compressor 10 and the ejector E5 and a decontamination liquid supply pipe LL5 communicating the hydrogen peroxide solution tank 20 and the ejector E5. The configuration and structure of the air supply pipe AL5 and the decontamination liquid supply pipe LL5 are the same as in the above first embodiment, and its description is herein omitted.

The secondary mist generation device M8 is composed of a mist receiving container and an ultrasonic atomizer and receives a primary mist containing a hydrogen peroxide solution conveyed from the ejector E5 to convert the same into a secondary mist and discharge the same into the inside of the room to be decontaminated R4. The structure and function of the mist receiving container and the ultrasonic atomizer will be described later. Also, the mist dispersion/diffusion device D5 (in FIG. 10, corresponding to "D5a and D5b") is disposed to be symmetrical with respect to the secondary mist generation device M8 to disperse and diffuse a hydrogen peroxide mist (secondary mist) discharged from the secondary mist generation device M8 into the inside of the room to be decontaminated R4 uniformly to the inside of the room to be decontaminated R4. The structure and function of the mist dispersion/diffusion device D5 will be described later.

In FIG. 10 illustrating this fourth embodiment, the secondary mist generation device M8 and the mist dispersion/diffusion device D5 are disposed in the center of an upper portion on one wall surface of the room to be decontaminated R4. In addition, the positions of the secondary mist generation device M8 and the mist dispersion/diffusion device D5 are not restricted thereto, and they may be disposed on other wall surface. Even in this case, the secondary mist generation device M8 and the mist dispersion/diffusion device D5 are preferably disposed at positions where the hydrogen peroxide mist can uniformly be dispersed and diffused into the inside of the room to be decontaminated R4.

The primary mist supply pipe ML4 communicates a discharge flow path of the ejector E5 and mist receiving container MR2 that constitutes the ultrasonic atomizer A2. The primary mist supply pipe ML4 is installed over a long distance from the vicinity of the air compressor 10 and the hydrogen peroxide solution tank 20 to the secondary mist generation device M8 disposed in the center of an upper portion on a wall surface of the room to be decontaminated R4. Herein, the material, diameter and conveyance distance of the primary mist supply pipe ML4 are the same as in the above first embodiment.

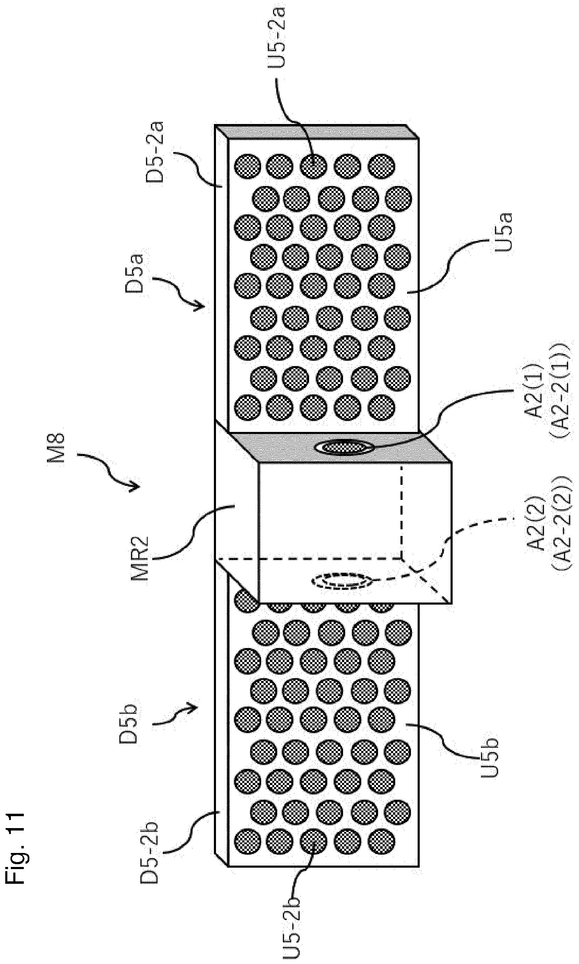

Subsequently, the secondary mist generation device M8 and the mist dispersion/diffusion device D5 will be described. FIG. 11 is a perspective view illustrating a first exemplary combination of a secondary mist generation device and a mist dispersion/diffusion device in this fourth embodiment. First, the secondary mist generation device M8 will be described. The mist dispersion/diffusion device D5 will be described later.

In FIG. 11, the secondary mist generation device M8 is composed of a mist receiving container MR2 and 2 ultrasonic atomizers A2(1), A2(2), which acts as a secondary mist generation means. The mist receiving container MR2 acts as a primary mist receiving container for receiving a primary mist containing a hydrogen peroxide solution conveyed from the ejector E5 and subjecting the same to gas-liquid separation. Also, the mist receiving container MR2 supplies the separated hydrogen peroxide solution to the ultrasonic atomizers A2 (1) , A2 (2) and discharges the separated air into the outside.

The ultrasonic atomizers A2 (1) , A2 (2) are each provided on 2 opposite surfaces of the secondary mist generation device M8 (in FIG. 10, on both right and left side surfaces) . The ultrasonic atomizers A2(1), A2(2) receive a hydrogen peroxide solution subjected to gas-liquid separation from the mist receiving container MR2, generate a fine hydrogen peroxide mist (secondary mist), and discharge the same into the inside of the room to be decontaminated R4. The ultrasonic atomizers A2(1), A2(2) discharge a hydrogen peroxide mist in the vertical direction from a disposed surface (in both right and left directions horizontally along a corner wall surface in FIG. 10).

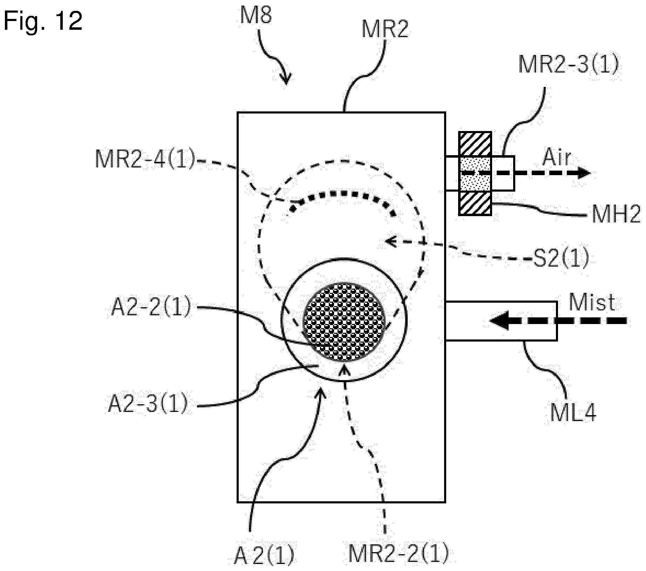

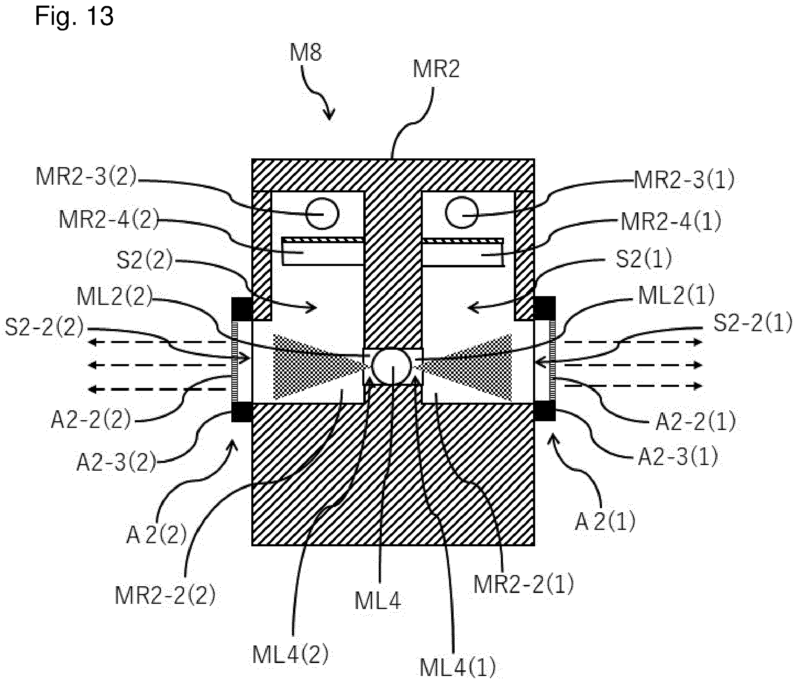

Herein, one exemplary secondary mist generation device M8 used in this fourth embodiment will be described. FIG. 12 is a right side view illustrating the secondary mist generation device used in this fourth embodiment seen from the side of a room to be decontaminated. FIG. 13 is a cross-sectional view illustrating the secondary mist generation device used in this fourth embodiment seen from the front in the room to be decontaminated.

In FIGS. 12 and 13, the mist receiving container MR2 constitutes internal spaces S2(1), S2(2) with a side internal portion having a semi-spindle-shaped cross section, and ultrasonic atomizers A2(1), A2(2) are attached to openings S2-2(1), S2-2(2) that are opened on an outer wall surface corresponding to a side lower end portion having a semi-spindle-shaped focusing width. A lower end portion of the internal space is provided with a focusing width to serve as liquid pools MR2-2(1), MR2-2(2) for a small amount of decontamination liquid subjected to gas-liquid separation. Also, an end of the primary mist supply pipe ML4 communicates with a rear central portion of the mist receiving container MR2 (at a position opposite the ultrasonic atomizer A2) toward the inside of the mist receiving container MR2, is internally divided into ML4(1), ML4(2) in 2 right and left directions, which are opened toward rear surfaces of the ultrasonic atomizers A2(1), A2(2), respectively (outside the room to be decontaminated).

Air vents MR2-3(1), MR2-3 (2) are opened at a rear upper end portion of the 2 internal spaces S2(1), S2 (2) of the mist receiving container MR2. Also, paths of the air vents MR2-3(1), MR2-3(2) may be provided with a filter MH2 resolving hydrogen peroxide. Baffle plates MR2-4(1), MR2-4(2) are provided between ends ML4(1), ML4(2) of the primary mist supply pipe ML4 of the 2 internal spaces S2(1), S2(2) of the mist receiving container MR2 and the air vents MR2-3(1), MR2-3(2).

The ultrasonic atomizers A2(1), A2(2) are composed of substantially annular disk-shaped perforated vibration plates A2-2(1), A2-2(2), each provided with a plurality of micropores (not shown) atomizing the decontamination liquid (hydrogen peroxide solution) subjected to gas-liquid separation, the micropores passing through the perforated vibration plate between the front surface and the back surface thereof, piezoelectric vibrators A2-3(1), A2-3(2) formed of a substantially annular disk in which the perforated vibration plates A2-2(1), A2-2(2) are subjected to film vibration, and a controller (not shown) controlling the vibration of the piezoelectric vibrators A2-3(1), A2-3(2). The perforated vibration plates A2-2(1), A2-2(2) are affixed to the piezoelectric vibrators A2-3(1), A2-3(2) so as to cover internal hole portions of the piezoelectric vibrators A2-3(1), A2-3(2).

In addition, the perforated vibration plates A2-2(1), A2-2(2) are attached such that the front surface faces the inside of the room to be decontaminated and the rear surface faces the liquid pools MR2-2(1), MR2-2(2) of the internal spaces S2(1), S2(2) of the mist receiving container MR2. Accordingly, a plurality of micropores of the perforated vibration plates A2-2(1), A2-2(2) pass through the inside of the room to be decontaminated R4 and the internal spaces S2(1), S2(2) of the mist receiving container MR2. In FIG. 11, the perforated vibration plates A2-2(1), A2-2(2) are disposed such that a hydrogen peroxide mist is discharged horizontally from the front surface of the perforated vibration plates A2-2(1), A2-2(2) of the ultrasonic atomizers A2(1), A2(2) and along the front surface of the mist dispersion/diffusion devices D5a, D5b.

In this state, the primary mist is discharged into the inside of the mist receiving container MR2 through ends ML4(1), ML4(2) of the primary mist supply pipe ML4. The rear surface of the perforated vibration plates A1-2(1), A1-2(2) and the ends ML4(1), ML4(2) of the primary mist supply pipe ML4 are opposite inside the mist receiving container MR1. Accordingly, the discharged primary mist is ejected directly onto the rear surface of the perforated vibration plates A2-2(1), A2-2(2) to be subjected to gas-liquid separation.

The separated decontamination liquid is converted into a fine hydrogen peroxide mist (secondary mist) through a plurality of micropores of the perforated vibration plates A2-2(1), A2-2 (2) under ultrasonic vibration to be discharged into the inside of the room to be decontaminated R4. Even if part of the decontamination liquid subjected to gas-liquid separation on the rear surface of the perforated vibration plates A2-2(1), A2-2(2) is retained in the liquid pools MR2-2(1), MR2-2(2), this is converted into a fine secondary mist, though in very small quantities, through a plurality of micropores of the perforated vibration plates A2-2(1), A2-2(2) to be discharged into the inside of the room to be decontaminated. Meanwhile, the separated air is discharged from the air vents MR2-3(1), MR2-3(2) into the outside . The diameter and number of the micropores of the perforated vibration plates A2-2(1), A2-2(2) are the same as in the above first embodiment, and its description is omitted.

Subsequently, the mist dispersion/diffusion devices D5a, D5b for dispersing and diffusing the hydrogen peroxide mist thus discharged into the inside of the room to be decontaminated will be described. In FIG. 11, a set of 2 mist dispersion/diffusion devices D5a, D5b are disposed so as to be symmetrical with respect to the secondary mist generation device M8. The two mist dispersion/diffusion devices D5a, D5b include ultrasonic vibration plates D5-2a, D5-2b, which are similar in structure, respectively.

One ultrasonic vibration plate D5-2a is disposed such that acoustic radiation pressure by ultrasonic vibration from the side in the discharge direction acts on the hydrogen peroxide mist discharged rightward in the figure horizontally from the ultrasonic atomizer A2(1). The other ultrasonic vibration plate D5-2b is disposed such that acoustic radiation pressure by ultrasonic vibration from the side in the discharge direction acts on the hydrogen peroxide mist discharged leftward in the figure from the ultrasonic atomizer A2(2). The structure and operation of the ultrasonic vibration plates D5-2a, D5-2b are the same as in the above first embodiment, and its description is omitted.

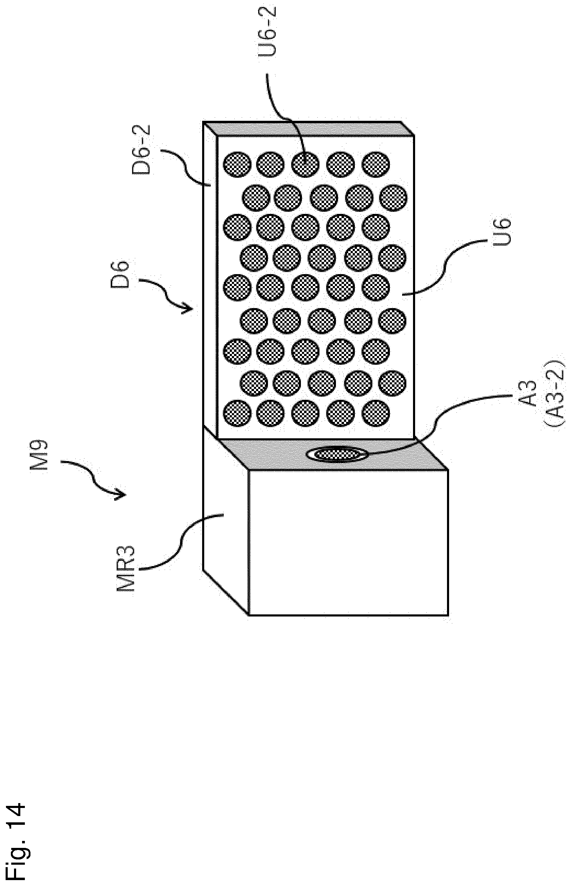

Also, the combination of a secondary mist generation device and a mist dispersion/diffusion device is not restricted to the above exemplary combination (see FIG. 11), and other example will be described as follows. FIG. 14 is a perspective view illustrating a second exemplary combination of a secondary mist generation device and a mist dispersion/diffusion device. In FIG. 14, one mist dispersion/diffusion device D6 is disposed for a secondary mist generation device M9. The secondary mist generation device M9 is composed of a mist receiving container MR3 and one ultrasonic atomizer A3, which acts as a secondary mist generation means. The structure and operation of the mist receiving container MR3 are almost the same as the above mist receiving container MR2. Also, the structure and operation of the ultrasonic atomizer A3 are the same as the above ultrasonic atomizer A2(1).

The mist dispersion/diffusion device D6 includes an ultrasonic vibration plate D6-2, which is similar to the above mist dispersion/diffusion device D5a. The ultrasonic vibration plate D6-2 is disposed such that acoustic radiation pressure by ultrasonic vibration from the side in the discharge direction acts on the hydrogen peroxide mist discharged rightward in the figure horizontally from the ultrasonic atomizer A3. The structure and operation of the ultrasonic vibration plates D6-2 are the same as in the above first embodiment, and its description is omitted.

Thus, in FIG. 14, the mist dispersion/diffusion device D6 is disposed only on one surface of the secondary mist generation device M9. Therefore, the secondary mist generation device M9 and the mist dispersion/diffusion device D6 are preferably disposed at an upper corner portion on a wall surface of the room to be decontaminated, rather than in the center of an upper portion thereon. Even in this case, a controller (not shown) controls the frequency, output, and transmission time of an ultrasonic transmitter U6-2 of the ultrasonic vibration plate D6-2, and the pressure on the hydrogen peroxide mist by acoustic radiation pressure can be changed in intermittent operation or stronger/weaker operation of ultrasonic transmission.

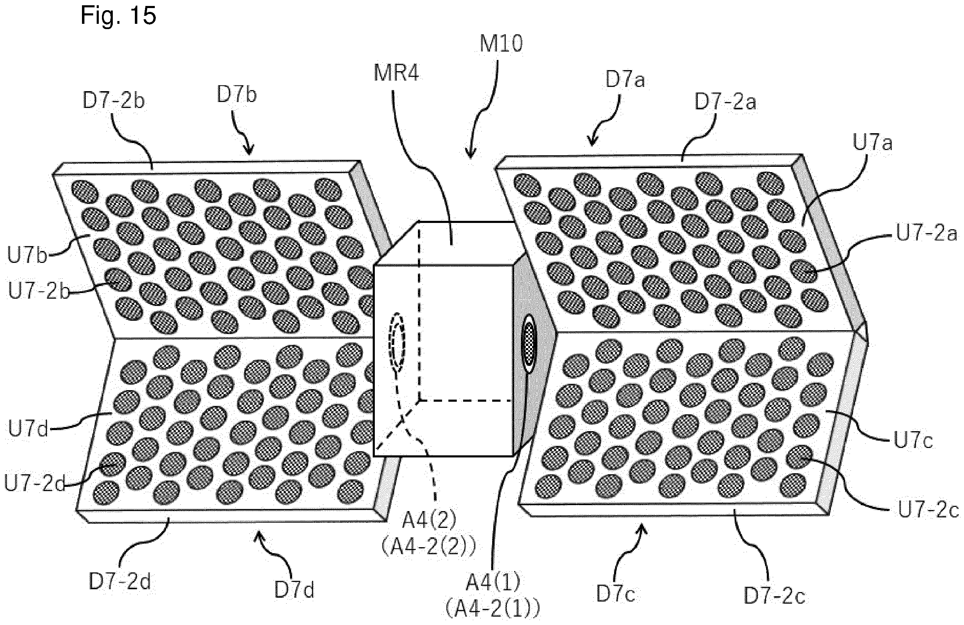

FIG. 15 is a perspective view illustrating a third exemplary combination of a secondary mist generation device and a mist dispersion/diffusion device. In FIG. 15, 2 sets of 2 mist dispersion/diffusion devices D7 (in FIG. 15, corresponding to "D7a, D7b, D7c, D7d") are each disposed to be symmetrical with respect to a secondary mist generation device M10.

On the right side of the secondary mist generation device M10 in the figure, mist dispersion/diffusion devices D7a, D7c are disposed such that surfaces of each of the devices slightly face and slant vertically in the figure. Meanwhile, on the left side of the secondary mist generation device M10 in the figure, mist dispersion/diffusion devices D7b, D7d are disposed such that surfaces of each of the devices slightly face and slant vertically in the figure.

The secondary mist generation device M10 is composed of a mist receiving container MR4 and 2 ultrasonic atomizers A4(1), A4(2), which acts as a secondary mist generation means . Also, the structure and operation of the mist receiving container MR4 and the ultrasonic atomizers A4(1), A4(2) are the same as the above mist receiving containers MR2 and the ultrasonic atomizers A2(1), A2(2).

The mist dispersion/diffusion devices D7a to D7d include ultrasonic vibration plates D7-2a to D7-2d, which are similar to the above mist dispersion/diffusion devices D5a, D5b. The structure and operation of the ultrasonic vibration plates D7-2a, D7-2d are the same as in the above first embodiment.

These ultrasonic vibration plates D7-2a to D7-2d are disposed such that acoustic radiation pressure by ultrasonic vibration acts on the hydrogen peroxide mist discharged both rightward and leftward (the direction along a wall surface of the room to be decontaminated) in the figure horizontally from the ultrasonic atomizers A4(1), A4(2). The ultrasonic vibration plate D7-2a, D7-2c are disposed such that acoustic radiation pressure by ultrasonic vibration acts on the hydrogen peroxide mist discharged rightward in the figure from the ultrasonic atomizer A4(1) so as to sandwich the hydrogen peroxide mist at a slight angle vertically in the lateral direction. Accordingly, the hydrogen peroxide mist is subjected to acoustic radiation pressure in the vertical direction and the resulting more directional property allows it to reach further swiftly.

Likewise, the ultrasonic vibration plates D7-2b, D7-2d are disposed such that acoustic radiation pressure by ultrasonic vibration acts on the hydrogen peroxide mist discharged leftward in the figure from the ultrasonic atomizer A4(2) so as to sandwich the hydrogen peroxide mist at a slight angle vertically in the lateral direction. Accordingly, the hydrogen peroxide mist is subjected to acoustic radiation pressure in the vertical direction and lateral direction and the resulting more directional property allows it to reach further swiftly.

Thus, in FIG. 15, 4 mist dispersion/diffusion devices D7a to D7d are disposed with angles in both right and left directions of the secondary mist generation device M10. Therefore, the secondary mist generation device M10 and the mist dispersion/diffusion devices D7a to D7d are preferably disposed in the center of the upper portion on the wall surface of the room to be decontaminated. Even in this case, a controller (not shown) controls the frequency, output, and transmission time of ultrasonic transmitters U7-2a to U7-2d of ultrasonic vibration plates D7-2a to D7-2d, and the pressure on the hydrogen peroxide mist by acoustic radiation pressure can be changed in intermittent operation or stronger/weaker operation of ultrasonic transmission.

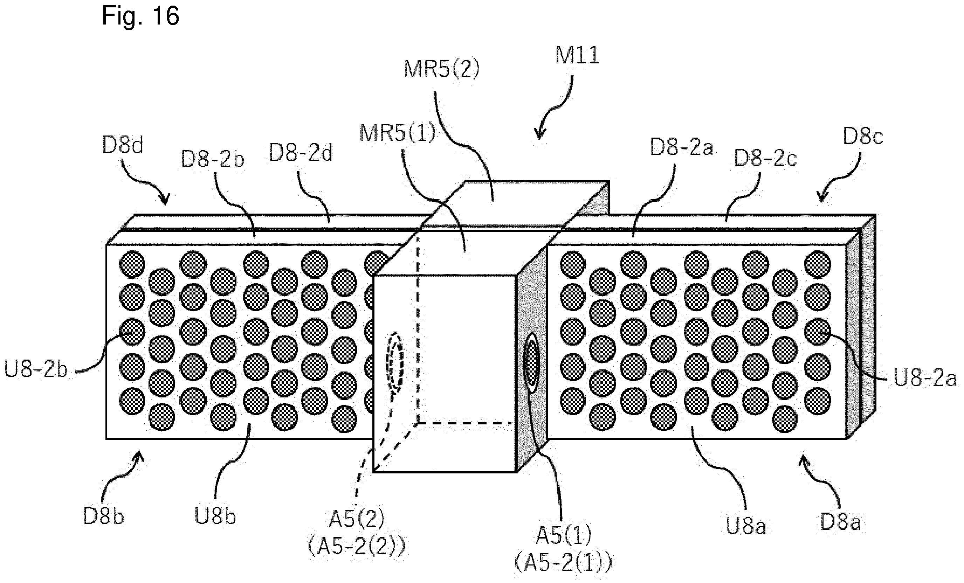

FIG. 16 is a perspective view illustrating a fourth exemplary combination of a secondary mist generation device and a mist dispersion/diffusion device. In FIG. 16, 2 sets of 2 mist dispersion/diffusion devices D8 (in FIG. 16, corresponding to "D8a, D8b, D8c, D8d") are each disposed to be symmetrical with respect to a secondary mist generation device M11. The set of 2 devices are disposed back-to-back. Specifically, the structure of the secondary mist generation device M11 and the mist dispersion/diffusion devices D8a to D8d in FIG. 16 is the same as that of the secondary mist generation device M8 and the 2 mist dispersion/diffusion devices D5a, D5b that are disposed back-to-back in FIG. 11.

The secondary mist generation device M11 is composed of 2 mist receiving containers MR5(1), MR5(2) and 4 ultrasonic atomizers A5(1) to A5(4) (in FIG. 16, the atomizers A5(3) and A5(4) are hidden on the rear surface side of the atomizers A5(1) and A5(2), respectively; not illustrated), which acts as a secondary mist generation means. Also, the structure and operation of the mist receiving container MR5(1), MR5(2) and the ultrasonic atomizers A5(1) to A5(4) are the same as the above mist receiving containers MR2 and the ultrasonic atomizers A2(1), A2(2).

The mist dispersion/diffusion devices D8a to D8d include ultrasonic vibration plates D8-2a to D8-2d, which are similar to the above mist dispersion/diffusion devices D5a, D5b. The structure and operation of the ultrasonic vibration plates D8-2a to D8-2d are the same as in the above first embodiment.

These ultrasonic vibration plates D8-2a to D8-2d are disposed such that acoustic radiation pressure by ultrasonic vibration acts on the hydrogen peroxide mist discharged both rightward and leftward in the figure horizontally from the ultrasonic atomizers A5(1) to A5(4). The ultrasonic vibration plate D8-2a is disposed such that acoustic radiation pressure by ultrasonic vibration in the lateral direction acts on the hydrogen peroxide mist discharged rightward on the front side in the figure from the ultrasonic atomizer A5(1). The ultrasonic vibration plate D8-2b is disposed such that acoustic radiation pressure by ultrasonic vibration in the lateral direction acts on the hydrogen peroxide mist discharged leftward on the front side in the figure from the ultrasonic atomizer A5(2).

Meanwhile, the ultrasonic vibration plate D8-2c is disposed such that acoustic radiation pressure by ultrasonic vibration in the lateral direction acts on the hydrogen peroxide mist discharged rightward on the rear side in the figure from the ultrasonic atomizer A5(3) (in FIG. 16, the atomizer A5(3) is hidden on the rear surface side of the atomizer A5(1); not illustrated). The ultrasonic vibration plate D8-2d is disposed such that acoustic radiation pressure by ultrasonic vibration in the lateral direction acts on the hydrogen peroxide mist discharged leftward on the rear side in the figure from the ultrasonic atomizer A5(4) (in FIG. 16, the atomizer A5(4) is hidden on the rear surface side of the atomizer A5(2); not illustrated).

Thus, in FIG. 16, 2 sets of 2 mist dispersion/diffusion devices D8a to D8d are each disposed back-to-back in both right and left directions of the secondary mist generation device M11. Therefore, the secondary mist generation device M11 and the mist dispersion/diffusion devices D8a to D8d are preferably disposed around the center of a ceiling wall, rather than in the center of an upper portion on a wall surface of the room to be decontaminated. Accordingly, 4 hydrogen peroxide mists discharged from the secondary mist generation device M11 are uniformly dispersed and diffused in all four directions from the center of the room to be decontaminated. Even in this case, a controller (not shown) controls the frequency, output, and transmission time of ultrasonic transmitters U8-2a to U8-2d of ultrasonic vibration plates D8-2a to D8-2d, and the pressure on the hydrogen peroxide mist by acoustic radiation pressure can be changed in intermittent operation or stronger/weaker operation of ultrasonic transmission.

An approach for decontaminating a room to be decontaminated R1 using the decontamination system 400 of this fourth embodiment is taken by disposing a secondary mist generation device M8 and a set of 2 mist dispersion/diffusion devices D5a, D5b illustrated in FIG. 11 in the center of an upper portion on a wall surface of a room to be decontaminated R4 (see FIG. 10). The capacity of the room to be decontaminated R4 and the amount of a hydrogen peroxide mist to be discharged into the room per unit time are the same as in the above first embodiment, and similarly favorable decontamination effects were obtained. Thus, description of the details of a decontamination method and decontamination effects is herein omitted.

### <Fifth embodiment>

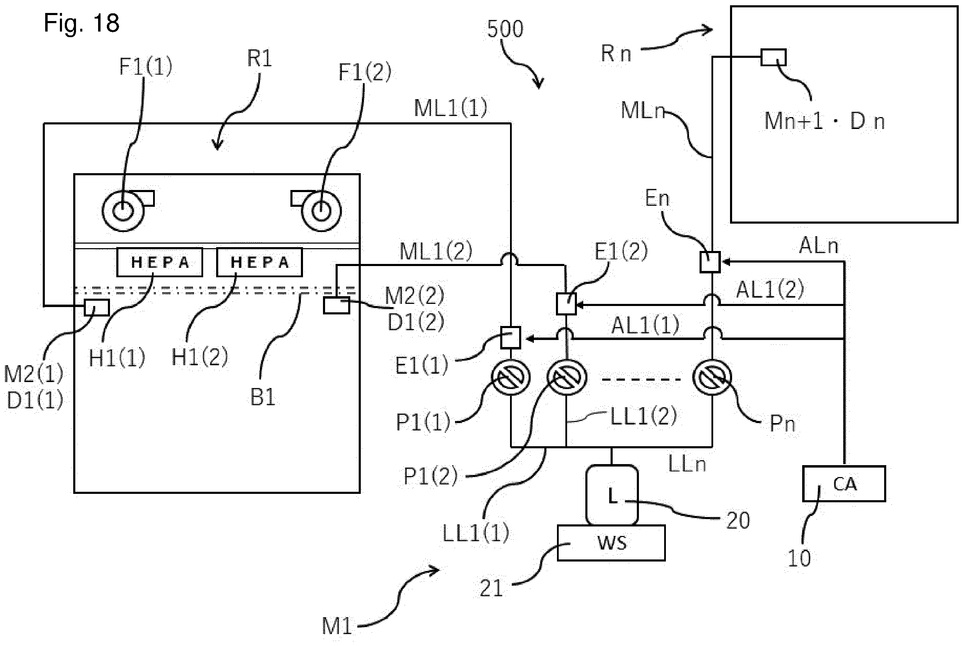

This fifth embodiment describes the case where a decontamination device of the present invention is disposed for a plurality of rooms to be decontaminated. FIG. 18 is a schematic block diagram illustrating a decontamination system according to a fifth embodiment of the present invention. As illustrated in FIG. 18, in a decontamination system 500 an isolator is composed of "n" rooms to be decontaminated (n is a positive integer), each denoted by R1 to Rn and having different space areas. Each room to be decontaminated, having a separate space, is provided at an upper portion therein with circulating fans F1 to Fn, HEPA filters H1 to Hn, and straightening plates B1 to Bn.

In this fifth embodiment, an isolator is composed of "n" rooms to be decontaminated, but the structure of an isolator is not restricted thereto, and the isolator may be a RABS, a clean room, a passing room, a pass box, or a combination thereof. There may also be one or more rooms to be decontaminated.

In FIG. 18, a decontamination system 500 includes an air compressor 10 and a hydrogen peroxide solution tank 20, which are shared by rooms to be decontaminated R1 to Rn. Also, the rooms to be decontaminated R1 to Rn include hydrogen peroxide mist generation devices M1 to Mn+1 (a primary mist generation device only, or a combination of a primary mist generation device and a secondary mist generation device), and mist dispersion/diffusion devices D1 to Dn, respectively.

In FIG. 18, one primary mist generation device M1 is shared by all the rooms to be decontaminated R1 to Rn (having ejectors E1 to En for the rooms to be decontaminated R1 to Rn, respectively), and the room to be decontaminated R1 is provided with 2 secondary mist generation devices M2(1), M2(2) and 2 mist dispersion/diffusion devices D1(1), D1(2). Meanwhile, the room to be decontaminated Rn has one secondary mist generation device Mn+1. This different configuration depends on the internal capacity of each room to be decontaminated.

The air compressor 10 acts as a compressed air generation means for generating compressed air, which is a carrier gas for conveying a hydrogen peroxide solution. The air compressor 10 is arranged so as to be separate from each of the rooms to be decontaminated R1 to Rn.

The hydrogen peroxide solution tank 20 acts as a decontamination liquid supply means for storing a hydrogen peroxide solution, which is the source of a hydrogen peroxide mist as a mist for decontamination. The hydrogen peroxide solution tank 20 is disposed near the air compressor 10 at a position separated from the rooms to be decontaminated R1 to Rn. Herein, the concentration of the hydrogen peroxide solution stored in the hydrogen peroxide solution tank 20 is not particularly restricted, and in general, it is preferably 30 to 35 % by weight in view of hazardous materials in use. Also, the hydrogen peroxide solution tank 20 includes a weighing device 21 for detecting the remaining amount of a hydrogen peroxide solution therein and a controller (not shown) for controlling the remaining amount.

The ejectors E1 to En of the primary mist generation device M1 act as a primary mist generation means for generating a primary mist by mixing a hydrogen peroxide solution with compressed air. The ejectors E1 to En are disposed adjacent to the air compressor 10 and the hydrogen peroxide solution tank 20 so as to be separate from each of the rooms to be decontaminated R1 to Rn.

The secondary mist generation devices M2 to Mn+1 are composed of mist receiving containers MR1 to MRn and ultrasonic atomizers A1 to An (both not illustrated in FIG. 18, but the structure is the same as in the above first embodiment). The mist receiving containers MR1 to MRn act as a primary mist receiving container for receiving a primary mist containing a hydrogen peroxide solution conveyed from the ejectors E1 to En and subjecting the mist to gas-liquid separation. Also, the mist receiving containers MR1 to MRn supply the separated hydrogen peroxide solution to the ultrasonic atomizers A1 to An and discharge the separated air into the outside. The structure of the mist receiving containers MR1 to MRn is the same as in the above first embodiment.

In FIG. 18 illustrating this fifth embodiment, the secondary mist generation devices M2 to Mn+1 are disposed above a work area of the rooms to be decontaminated R1 to Rn, respectively (beneath the straightening plates B1 to Bn, respectively).

The ultrasonic atomizers A1 to An receive a hydrogen peroxide solution subjected to gas-liquid separation from the mist receiving containers MR1 to MRn, generate a fine secondary mist, and discharge the same into the inside of the rooms to be decontaminated R1 to Rn. The ultrasonic atomizers A1 to An constitute the secondary mist generation devices M2 to Mn+1 acting as the secondary mist generation means, together with the mist receiving containers MR1 to MRn. The structure of the ultrasonic atomizers A1 to An is the same as in the above first embodiment.

The mist dispersion/diffusion devices D1 to Dn are disposed adjacent to a lower portion of the secondary mist generation devices M2 to Mn+1. The mist dispersion/diffusion devices D1 to Dn each include an ultrasonic vibration plate as in the above first embodiment, and acoustic radiation pressure by ultrasonic vibration from a lower portion of a discharge port acts on the hydrogen peroxide mist discharged horizontally from the ultrasonic atomizers A1 to An. The structure and operational advantage of the mist dispersion/diffusion devices D1 to Dn are the same as in the above first embodiment.

The largest room to be decontaminated R1, as described above, is provided with 2 ejectors E1(1), E1(2), 2 secondary mist generation devices M2(1), M2(2), and 2 mist dispersion/diffusion devices D1(1), D1(2)2. This is because that the decontamination efficiency can be higher by discharging a hydrogen peroxide mist from 2 locations into the large-volume room to be decontaminated R1. Also, more secondary mist generation devices and mist dispersion/diffusion devices may be provided for one room, depending on the space area of the room to be decontaminated. Accordingly, even in cases where a plurality of secondary mist generation devices and mist dispersion/diffusion devices is provided for one room, a primary mist supply pipe can be small in diameter, thereby saving equipment costs.

In FIG. 18, a decontamination system 500 includes air supply pipes AL1 to ALn communicating an air compressor 10 and ejectors E1 to En, decontamination liquid supply pipes LL1 to LLn communicating a hydrogen peroxide solution tank 20 and the ejectors E1 to En, and primary mist supply pipes ML1 to MLn communicating the ejectors E1 to En and mist receiving containers MR1 to MRn.

The air supply pipes AL1 to ALn communicate a discharge port of the air compressor 10 and a driving flow path (not shown) of each of the ejectors E1 to En. Conduit lines of the air supply pipes AL1 to ALn are each provided with an on-off valve (not shown) controlling the supply of compressed air. Herein, the material and diameter of the air supply pipes AL1 to ALn are not particularly restricted, and as in the above first embodiment, such a pipe is preferably a stainless pipe having an internal diameter of 1 to 10 mm. A conduit line between the air compressor 10 and each of the air supply pipes AL1 to ALn may be provided with an air dryer, an air regulator, an auto-drain, an oil mist separator, and other filter (each not shown in FIG. 18).

Each of the decontamination liquid supply pipes LL1 to LLn communicates a supply port of the hydrogen peroxide solution tank 20 and a suction flow path (not shown) of each of the ejectors E1 to En. Conduit lines of the decontamination liquid supply pipes LL1 to LLn are provided with tube pumps P1 to Pn controlling the supply of a hydrogen peroxide solution, respectively. Herein, the material and diameter of the decontamination liquid supply pipes LL1 to LLn are not particularly restricted so long as they can serve for a hydrogen peroxide solution, and as in the above first embodiment, such a pipe is preferably a stainless pipe having an internal diameter of 1 to 10 mm.

The primary mist supply pipes ML1 to MLn communicate a discharge flow path of each of the ejectors E1 to En and mist receiving containers MR1 to MRn that constitute the ultrasonic atomizers A1 to An. The primary mist supply pipes ML1 to MLn are installed over a long distance from the vicinity of the air compressor 10 and the hydrogen peroxide solution tank 20 to the secondary mist generation devices M2 to Mn+1 disposed inside an upper wall of each of the rooms to be decontaminated R1 to Rn. Herein, the material and diameter of the primary mist supply pipes ML1 to MLn may preferably be determined so long as a required amount of hydrogen peroxide mist can be conveyed over a long distance per unit time, and as in the above first embodiment, such a pipe is preferably a stainless pipe having an internal diameter of 1 to 10 mm.

Thus, a hydrogen peroxide mist can separately be discharged into each room to be decontaminated to accurately decontaminate each room by disposing the air supply pipes AL1 to ALn, the decontamination liquid supply pipes LL1 to LLn and the primary mist supply pipes ML1 to MLn in the rooms to be decontaminated R1 to Rn.

As shown in FIG. 18, the conveyance distance of each of the primary mist supply pipes ML1 to MLn is longer than the conveyance distance of each of the air supply pipes AL1 to ALn or the conveyance distance of each of the decontamination liquid supply pipes LL1 to LLn. The conveyance distance for the primary mist by each of the primary mist supply pipes ML1 to MLn is not particularly restricted, and normally is 3 to 100 m or so. Meanwhile, the conveyance distance of each of the air supply pipes AL1 to ALn or the conveyance distance of each of the decontamination liquid supply pipes LL1 to LLn can be shortened.

In this fifth embodiment, the primary mist is a high-density mixing gas/liquid of compressed air and hydrogen peroxide solution with a high conveyance speed, and primary mist supply pipes ML1 to MLn used may each be a small-diameter pipe. Therefore, the rooms to be decontaminated can be provided with long primary mist supply pipes ML1 to MLn. Accordingly, large-scale equipment such as large-diameter ducts is not required.

Also, a hydrogen peroxide solution in a primary mist is present as a liquid, thereby requiring no warming of primary mist supply pipes ML1 to MLn to prevent condensation. Therefore, even in cases where a long pipe is installed for each room to be decontaminated, large-scale equipment such as anti-condensation heaters is not required.

Thus, the shorter conveyance distance of each of the decontamination liquid supply pipes LL1 to LLn can accurately determine the amount of a hydrogen peroxide solution supplied to each of the ejectors E1 to En. Accordingly, the amount of the hydrogen peroxide solution supplied to the mist receiving containers MR1 to MRn for each room to be decontaminated can accurately be determined, and the amount of a hydrogen peroxide mist discharged into the room to be decontaminated is clearly determined. Meanwhile, the hydrogen peroxide solution in the primary mist, which is present as a liquid, is not condensed, thereby conveying a hydrogen peroxide solution over a long distance and accurately by elongating the conveyance distance of the primary mist supply pipes ML1 to MLn. Moreover, complete conveyance of the hydrogen peroxide solution in the primary mist supply pipes ML1 to MLn by compressed air allows no residual dead liquid to stay in the pipe.

An approach for decontaminating rooms to be decontaminated R1 to Rn using the decontamination system 500 of this fifth embodiment is the same as in the above first embodiment for each of the rooms to be decontaminated R1 to Rn, and its description is herein omitted.

As clearly described in each of the above embodiments, the present invention can provide a decontamination system capable of achieving a decontamination effect with a proper amount of hydrogen peroxide mist supplied to a room to be decontaminated by further refining a hydrogen peroxide mist supplied to the inside of the room to be decontaminated for dispersion/diffusion, and reducing the duration of operations such as aeration to achieve more efficient decontamination works.

The present invention is achieved not only by each of the above embodiments, but also by the following various alternatives.
(1) In each of the above embodiments, the compressed air generation means employed is an air compressor, but the means is not restricted thereto, and other means such as a high pressure air cylinder may be used.
(2) In each of the above embodiments, the primary mist generation means employed is an ejector, but the means is not restricted thereto, and other gas-liquid mixing means such as a gas-liquid pump like a two-fluid spray nozzle may be used.
(3) In each of the above embodiments, a tube pump employed is a conduit line of a decontamination liquid supply pipe, but the tube pump is not restricted thereto, and any other pump or liquid supply means may be used.

### REFERENCE SIGNS LIST

100, 200, 300, 400, 500...Decontamination system,
10...Air compressor, AL1 to ALn...Air supply pipe,
20...Hydrogen peroxide solution tank, 21...Weighing device, LL1 to LLn...Decontamination liquid supply pipe,
P1 to Pn...Tube pump, E1 to En...Ej ector,
ML1 to MLn...Primary mist supply pipe,
M1 to Mn...Hydrogen peroxide mist generation device (Primary mist generation device, Secondary mist generation device), MR1 to MRn...Mist receiving container, MR1-2, MR2-2...Liquid pool,
MR1-3, MR2-3...Air vent, MR1-4, MR2-4...Baffle plate,
S1, S2...Internal space, S1-2, S2-2...Opening,
MH1, MH2...Hydrogen peroxide decomposition filter,
A1 to An...Ultrasonic atomizer, A1-2 , A2-2...Perforated vibration plate,
A1-3, A2-3...Piezoelectric vibrator,
D1 to Dn , D5a , D5b , D7a to D7d , D8a to D8d...Mist dispersion/diffusion device,
D1a , D5-2a , D5-2b , D6-2 , D7-2a to D7-2d , D8-2a to D8-2d...Ultrasonic vibration plate,
U1, U5a, U5b, U6, U7a to U7d, U8a to U8d...Base,
U1a , U5-2a , U5-2b , U6-2 , U7-2a to U7-2d , U8-2a to U8-2d...Ultrasonic transmitter,
R1 to Rn...Room to be decontaminated, F1 to Fn...Circulating fan, H1 to Hn...HEPA filter,
B1 to Bn...Straightening plate,
R1 to R4...Room to be decontaminated, X1 to X5...Mist discharge port.

When one room has two units, each unit is numbered as (1) or (2).

## Claims

1. A decontamination system comprising
a mist generation means for generating a mist for decontamination by converting a decontamination liquid into a mist, a mist discharge port for discharging the mist for decontamination into the inside of a room to be decontaminated, and a mist dispersion/diffusion means for dispersing and diffusing the mist for decontamination discharged, wherein
the mist discharge port is opened on an internal side wall surface of the room to be decontaminated to discharge the mist for decontamination into the inside of the room to be decontaminated, and
the mist dispersion/diffusion means comprises a vibration plate disposed adjacent to a lower portion of the mist discharge port on an internal side wall surface of the room to be decontaminated or adjacent to a side surface in a mist discharge direction, the vibration plate is subjected to ultrasonic vibration to generate sound flows from a plate surface by an ultrasound in the vertical direction, and the mist for decontamination discharged from the mist discharge port is pressed by acoustic radiation pressure backward or laterally in stationary operation, intermittent operation or stronger/weaker operation to thoroughly disperse and diffuse the mist for decontamination entirely inside the room to be decontaminated.

2. The decontamination system according to claim 1, wherein
the mist generation means comprises a decontamination liquid supply unit for storing a decontamination liquid and supplying the same through a decontamination liquid supply pipe and a compressed air supply device for generating compressed air and supplying the same through an air supply pipe to generate the mist for decontamination from the supplied decontamination liquid and compressed air.

3. The decontamination system according to claim 1, wherein
the mist generation means comprises a primary mist generation means and a secondary mist generation means,
the primary mist generation means comprises a decontamination liquid supply unit for storing a decontamination liquid and supplying the same through a decontamination liquid supply pipe and a compressed air supply device for generating compressed air and supplying the same through an air supply pipe to generate a primary mist from the supplied decontamination liquid and compressed air and supply the same to the secondary mist generation means through a primary mist supply pipe,
the secondary mist generation means comprises a primary mist receiving container for subjecting the primary mist supplied to gas-liquid separation and an ultrasonic atomizer, and the decontamination liquid subjected to gas-liquid separation is converted into a fine, atomized secondary mist to be supplied to the mist discharge port, and
the mist discharge port discharges the secondary mist into the inside of the room to be decontaminated as the mist for decontamination.

4. The decontamination system according to any one of claims 1 to 3, wherein
the mist for decontamination supplied to the inside of the room to be decontaminated is further refined by ultrasonic vibration generated from the ultrasonic vibration plate.

5. The decontamination system according to claim 3, wherein
the primary mist generation means, which comprises the decontamination liquid supply unit and the compressed air supply device, is shared by a plurality of rooms to be decontaminated, and
each of the rooms to be decontaminated comprises the secondary mist generation means, the mist discharge port and the mist dispersion/diffusion means.

6. The decontamination system according to claim 5, wherein
the decontamination liquid supply unit, the compressed air supply device and the primary mist generation means are arranged so as to be separate from each of the rooms to be decontaminated through the primary mist supply pipe,
the secondary mist generation means is arranged adjacent to a corresponding room to be decontaminated or indoor through the primary mist supply pipe, whereby
the conveyance distance of the primary mist supply pipe to each room to be decontaminated is longer than the conveyance distance of the decontamination liquid supply pipe corresponding to the room to be decontaminated.

7. The decontamination system according to any one of claims 3, 5, and 6, wherein
the ultrasonic atomizer comprises a piezoelectric vibrator and a perforated vibration plate provided with a plurality of micropores for atomizing the decontamination liquid subjected to gas-liquid separation by vibration of the piezoelectric vibrator, the micropores passing through the perforated vibration plate between the front surface and the back surface thereof,
the ultrasonic atomizer is disposed such that the front surface of the perforated vibration plate faces the inside of the room to be decontaminated as the mist discharge port and the rear surface faces the inside of the primary mist receiving container, and
the primary mist supplied to the primary mist receiving container is ejected from the primary mist supply pipe onto the rear surface of the perforated vibration plate to be subjected to gas-liquid separation, and is atomized when the separated decontamination liquid moves from the rear surface to the front surface of the perforated vibration plate to be discharged into the inside of the room to be decontaminated with the front surface serving as the mist discharge port.

8. The decontamination system according to any one of claims 3, 5, and 6, wherein
the ultrasonic atomizer comprises a piezoelectric vibrator and a perforated vibration plate provided with a plurality of micropores for atomizing the decontamination liquid subjected to gas-liquid separation by vibration of the piezoelectric vibrator, the micropores passing through the perforated vibration plate between the front surface and the back surface thereof,
the ultrasonic atomizer is disposed such that the front surface of the perforated vibration plate faces the inside of the room to be decontaminated as the mist discharge port and the rear surface faces a liquid pool provided at an internal lower end portion of the primary mist receiving container, and
the primary mist supplied to the primary mist receiving container is discharged from the primary mist supply pipe into the inside of the primary mist receiving container to be subjected to gas-liquid separation, and is atomized after the separated decontamination liquid is collected at the liquid pool of the primary mist receiving container and moves from the rear surface to the front surface of the perforated vibration plate to be discharged into the inside of the room to be decontaminated with the front surface serving as the mist discharge port.
